# EUROPEAN PATENT APPLICATION

(11) **EP 4 435 109 A1**
(43) Date of publication of application: **25.09.2024**
(21) Application number: 22895653.8
(22) Date of filing: 16.11.2022
(51) Int. Cl.: C12N 15/85, A01K 67/027, C12N 5/10, C12P 21/00

(54) **PROMOTER ACTIVATING SEQUENCE, EXPRESSION VECTOR COMPRISING SAID PROMOTER ACTIVATING SEQUENCE, AND MAMMALIAN CELL COMPRISING SAID EXPRESSION VECTOR**

(30) Priority: 16.11.2021 JP 2021186122
(71) Applicant: Tokyo University of Pharmacy and Life Science, Hachioji-shi, Tokyo 192-0392 (JP); National University Corporation Tottori University, Tottori-shi, Tottori 680-8550 (JP)
(72) Inventor: TOMIZUKA, Kazuma, Hachioji-shi, Tokyo 192-0392 (JP); UNO, Narumi, Hachioji-shi, Tokyo 192-0392 (JP); KAZUKI, Yasuhiro, Hachioji-shi, Tokyo 192-0392 (JP)
(74) Representative: Mewburn Ellis LLP
(86) International application number: PCT/JP2022/042594
(87) International publication number: WO 2023/090372

(57) **Abstract**

An object of the present invention is to provide a promoter activation sequence that allows for stable and efficient expression of a DNA of interest in a mammalian cell. The present invention provides a promoter activation sequence comprising a nucleotide sequence represented by SEQ ID NO: 1, or a nucleotide sequence of 850 or more in a nucleotide sequence having 85% or higher sequence identity thereto; an expression vector comprising the promoter activation sequence, a promoter, and a DNA of interest; and a mammalian cell or a mammal comprising the expression vector.

## Description

### Technical Field

The present invention relates to a nucleotide sequence that can activate a promoter, an expression vector comprising the promoter activation sequence, and a mammalian cell comprising the expression vector. The present invention further relates to a method for efficiently producing a protein intended to be expressed using the mammalian cell.

### Background Art

Techniques of producing bio-pharmaceuticals using mammalian cells or techniques of gene therapy have made substantial progress along with advances in biotechnology in recent years. For practical use of such techniques, it is very important to stably highly express a gene intended to be expressed (hereinafter, referred to as a gene of interest).

However, when a foreign protein is produced by transferring and expressing the gene of interest, epigenetic modification such as methylation or structural change in host chromosomes such as heterochromatinization is known to occur, thereby causing the transferred gene of interest to undergo inactivation or suppression of expression or a phenomenon of gene silencing to occur. Such a phenomenon disadvantageously makes it difficult to regulate the expression of the transferred gene of interest.

In order to cope with such problems, a promoter sequence of a housekeeping gene, which is commonly expressed in a given amount among many tissues or cells and is involved in the maintenance of cell function, is used as a promoter for the expression of the gene of interest. However, sufficient expression of the gene of interest may still not be obtained even if the promoter sequence of the housekeeping gene is used. Hence, it is desired to identify a nucleotide sequence that has a function of activating a promoter and allows for stable high expression of the gene of interest transferred to a mammalian cell (hereinafter, referred to as a promoter activation sequence).

Human ubiquitin C (hereinafter, abbreviated to UbC) gene is a housekeeping gene. The UbC gene is widely expressed in somatic cells including fibroblasts. A promoter sequence of the UbC gene is known to reside in a region of approximately 1.3 kb on the 5' side from a UbC gene initiation codon, and is generally used for the purpose of expressing the gene of interest in animal cells.

Non Patent Literature 1 and Patent Literature 1 disclose a DNA fragment of approximately 15 kb positioned upstream of the human UbC gene promoter. These literatures further show that the activity of the UbC gene promoter is enhanced by adding this DNA fragment of approximately 15 kb in constructing a vector.

However, the DNA fragment of Non Patent Literature 1 and Patent Literature 1, because of being as large as approximately 15 kb, is often accompanied by technical difficulties in performing gene manipulation, and disadvantageously has limitations on their scopes of use. In addition, this DNA fragment of approximately 15 kb is derived from the upstream genome of the UbC gene and has therefore been considered ineffective for generally used promoters other than the UbC gene promoter.

As for other sequences having an anti-silencing effect, ubiquitous chromatin operating elements (UCOE) derived from a human HNRPA2B1-CBX gene region most widely used have been reported to have anti-silencing activity.

As one example thereof, Non Patent Literature 2 discloses that a sequence of 1,5 k bp (A2UCOE) having anti-silencing activity was identified. Non Patent Literature 2 also discloses that A2UCOE and a fragment thereof exhibited an anti-silencing effect on the activity of spleen focus forming virus (SFFV) promoter.

Non Patent Literature 3 and Patent Literature 2 disclose that SURF-UCOE was identified as an anti-silencing sequence of approximately 1 kb derived from a human SURF 1-SURF2 gene region. Non Patent Literature 3 and Patent Literature 2 further disclose that SURF-UCOE and a partial sequence thereof exhibit anti-silencing against the activity of EF1α, PGK, CMV, and RSV promoters.

Specifically, the disclosure of Non Patent Literature 2, Non Patent Literature 3, and Patent Literature 2 is related to suppressed silencing of a promoter linked to UCOE in a human gene region. However, since UCOE contains an endogenous promoter sequence, protein-encoding exons, and introns, transcription from the endogenous promoter contained in the UCOE or the presence of the exons or the introns might produce unexpected results in expressing the gene of interest under the control of a predetermined promoter.

Non Patent Literature 4 discloses that D424 which is a microsatellite sequence of 3.3 kb located in a human chromosome 4 telomere region, and a partial sequence thereof suppress the silencing of human EF1α promoter activity. The D424 and the partial sequence thereof described above contain the open reading frame (ORF) of DUX gene. Therefore, in the case of expressing a gene by the addition of the D424 and the partial sequence thereof, a protein encoded by the ORF might be expressed.

### Citation List

### Patent Literature

Patent Literature 1: International Publication No. WO2011/071147
Patent Literature 2: International Publication No. WO2020/223160

### Non Patent Literature

Non Patent Literature 1: Kakeda et al., Biochemical and Biophysical Research Communications, 415 439-444 (2011)
Non Patent Literature 2: Kunkel et al., Scientific Reports, [7-7919], (2017)
Non Patent Literature 3: Rudina et al., BioRxiv, (2019), Doi: 10.1101/626713
Non Patent Literature 4: Rival-Gervier et al., Molecular Therapy, vol. 21, 1536-1550, (2013)

### Summary of Invention

### Technical Problem

An object of the present invention is to provide a novel approach capable of stably highly expressing a gene of interest. More specifically, an object of the present invention is to obtain a promoter activation sequence that is an upstream genomic sequence of human UbC gene promoter and has a smaller size than that of a promoter activation sequence of Patent Literature 1 and Non Patent Literature 1.

### Solution to Problem

As a result of conducting diligent studies, the present inventors have found that a DNA fragment comprising the nucleotide sequence represented by SEQ ID NO: 1 activates a promoter. The present inventors have further prepared an expression vector comprising the promoter activation sequence, a promoter, and a gene of interest and prepared a mammalian cell harboring the expression vector. The present inventors have then found that in the mammalian cell, silencing is suppressed and stable high expression of the gene of interest can be obtained, leading to the completion of the present invention.

Thus, the present invention has the following features (1) to (17).
(1) A promoter activation sequence comprising a nucleotide sequence represented by SEQ ID NO: 1 or a nucleotide sequence of 850 or more in a nucleotide sequence having 85% or higher sequence identity thereto.
(2) An expression vector comprising a promoter activation sequence according to (1), a promoter, and a DNA of interest.
(3) The expression vector according to (2), wherein the promoter is a constitutive expression promoter.
(4) The expression vector according to (3), wherein the constitutive expression promoter is selected from the group consisting of EF1α promoter, PGK promoter, and UBC promoter.
(5) The expression vector according to any of (2) to (4), wherein the promoter activation sequence according to (1) is comprised at 5' upstream of the promoter.
(6) The expression vector according to any of (2) to (5), wherein the expression vector is a mammalian artificial chromosome vector.
(7) The expression vector according to (6), wherein the expression vector is a human artificial chromosome vector.
(8) A mammalian cell carrying the expression vector according to any of (2) to (7).
(9) The cell according to (8), wherein the animal cell is a human cell or a CHO cell.
(10) The cell according to (9), wherein the human cell is a human pluripotent stem cell or a human somatic stem cell.
(11) The cell according to (10), wherein the human pluripotent stem cell is a human iPS cell or a human ES cell, and the human somatic stem cell is a human MSC cell.
(12) The cell according to any of (8) to (11), wherein owing to the presence of the promoter activation sequence according to (1), the cell exhibits stable high expression of the DNA of interest.
(13) The cell according to (12), wherein the stable high expression of the DNA of interest is brought about by expression enhancing activity and/or anti-silencing activity against the DNA of interest.
(14) A nonhuman mammal comprising the expression vector according to any of (2) to (7).
(15) A method for producing a protein encoded by a DNA of interest, comprising culturing the mammalian cell according to any of (8) to (13) in a medium for animal cell culture, and collecting the protein encoded by the DNA of interest from a culture product obtained by the culture.
(16) A method for suppressing the silencing of a DNA of interest in a cell by transferring the expression vector according to any of (2) to (7) to the cell.
(17) A method for enhancing the expression of a DNA of interest in a cell by transferring the expression vector according to any of (2) to (7) to the cell.

The present specification encompasses the contents disclosed in Japanese Patent Application No. 2021-186122 on which the priority of the present application is based.

### Advantageous Effects of Invention

The present invention provides a promoter activation sequence comprising a nucleotide sequence consisting of 850 or more in the nucleotide sequence represented by SEQ ID NO: 1. The present invention further provides an expression vector comprising the promoter activation sequence, a promoter, and a gene of interest. Stable and efficient expression of the gene of interest is achieved in a mammalian cell harboring the expression vector.

### Brief Description of Drawings

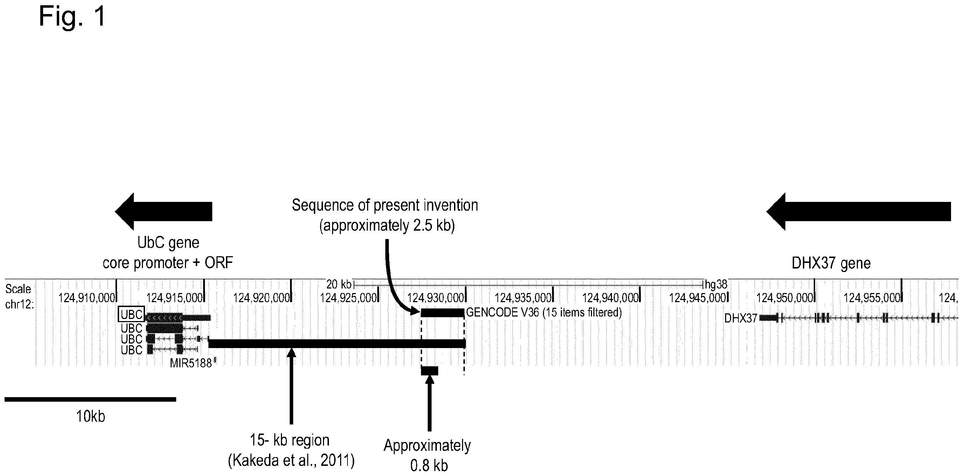

[Figure 1] Figure 1 shows a gene map in the vicinity of the sequence of the present application (SEQ ID NO: 1: approximately 2.5 kb). This gene map shows a region from 124,910,000 to 124,955,000 of human chromosome 12 obtained from the USCC genome browser. The bar line indicated by the term "Sequence of present application (approximately 2.5 bp)" depicts a region corresponding to the nucleotide sequence represented by SEQ ID NO: 1 (2549 bp). The bar line indicated by the term "Approximately 0.8 bp)" depicts a region corresponding to a nucleotide sequence of approximately 0.8 bp used in Examples described below in SEQ ID NO: 1 (2549 bp). The left-pointing arrows depict regions containing UbC gene (left) and DHX37 gene (right) in chromosome 12. The left bar line indicated by the term " 15-kb region" depicts a region corresponding to a nucleotide sequence of 15 kb disclosed in Patent Literature 1 and Non Patent Literature 1.

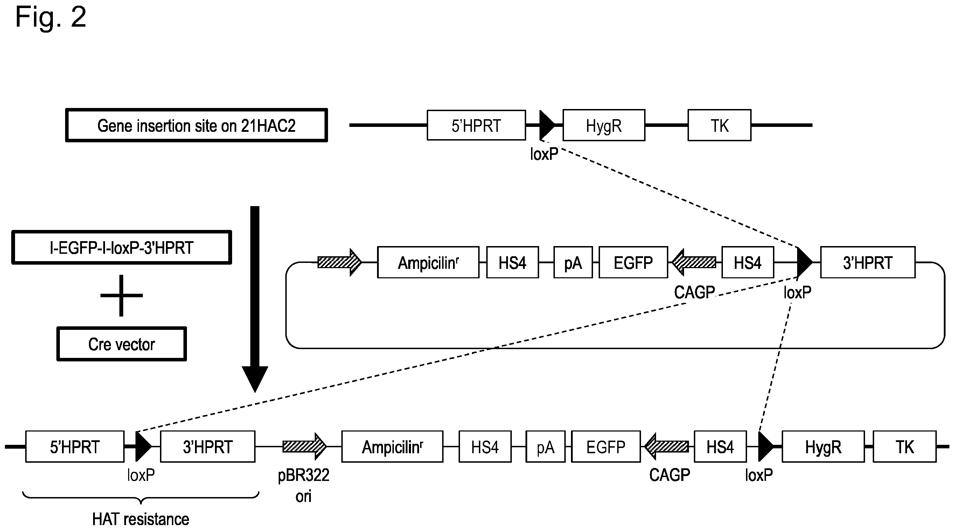

[Figure 2] Figure 2 is a diagram showing a scheme to prepare a HAT-resistant clone with one copy of an expression unit inserted in 21HAC2.

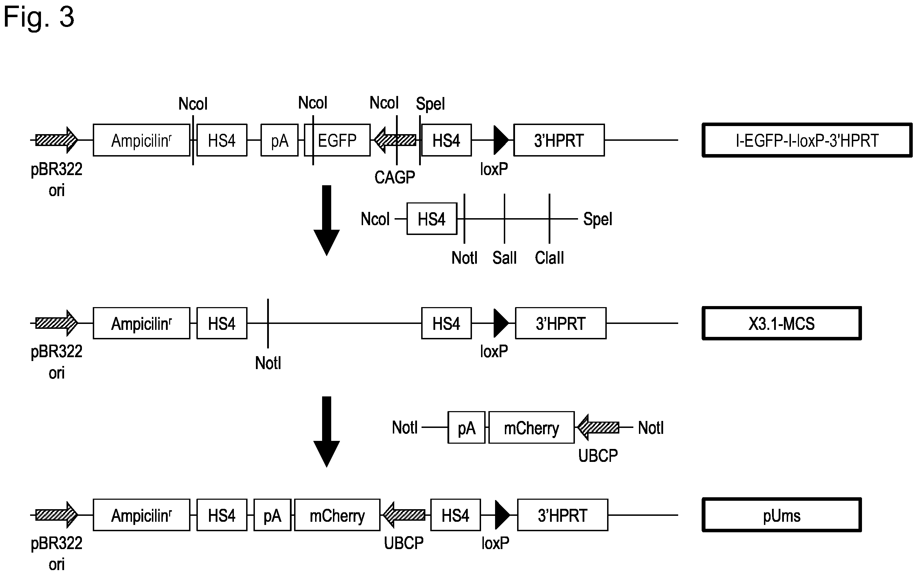

[Figure 3] Figure 3 is a diagram showing a scheme to construct a red fluorescent protein (mCherry) expression vector X3.1 -UbCP-mCherry-SV40pA (pUms vector) for human artificial chromosome (HAC) transfer.

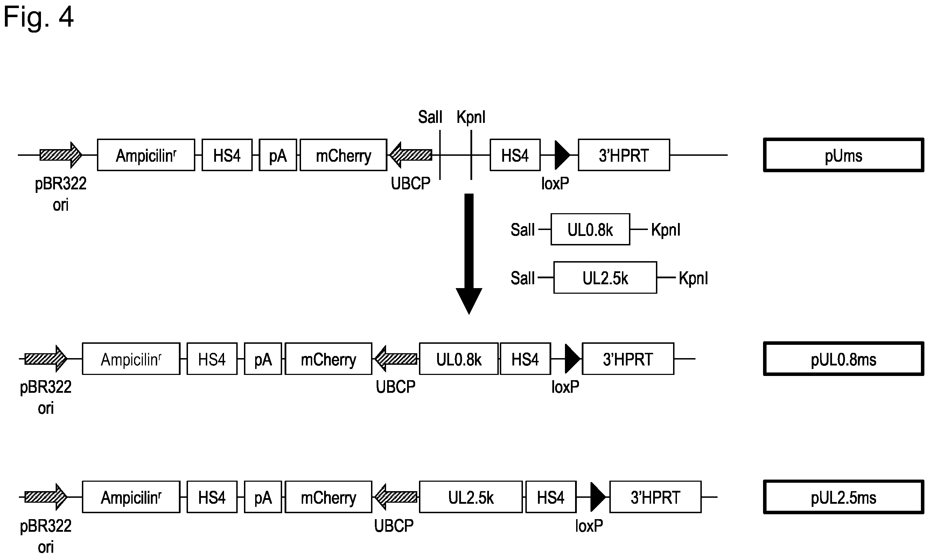

[Figure 4] Figure 4 is a diagram showing a scheme to construct pUL0.8ms vector and pUL2.5ms vector with UbC gene upstream genome added to pUms vector.

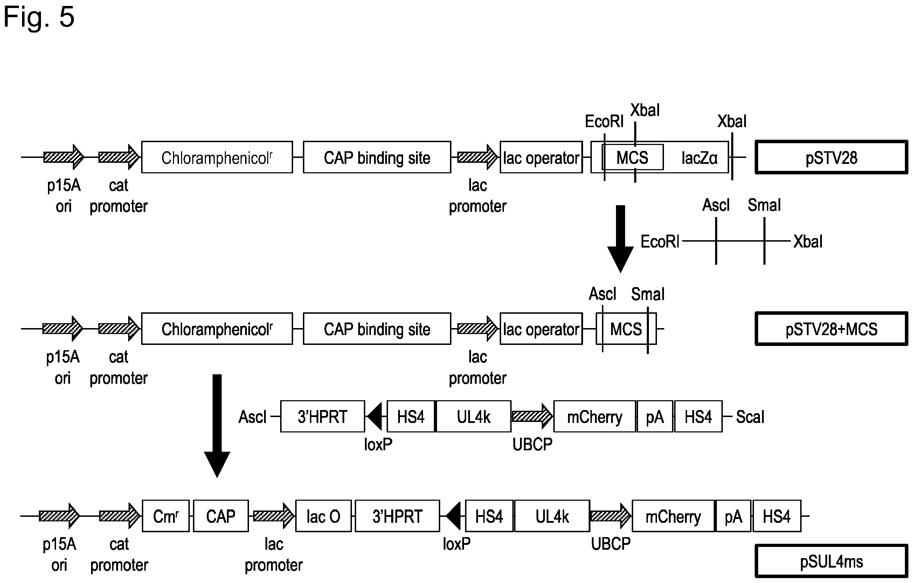

[Figure 5] Figure 5 is a diagram showing a scheme to construct pSUL4ms vector.

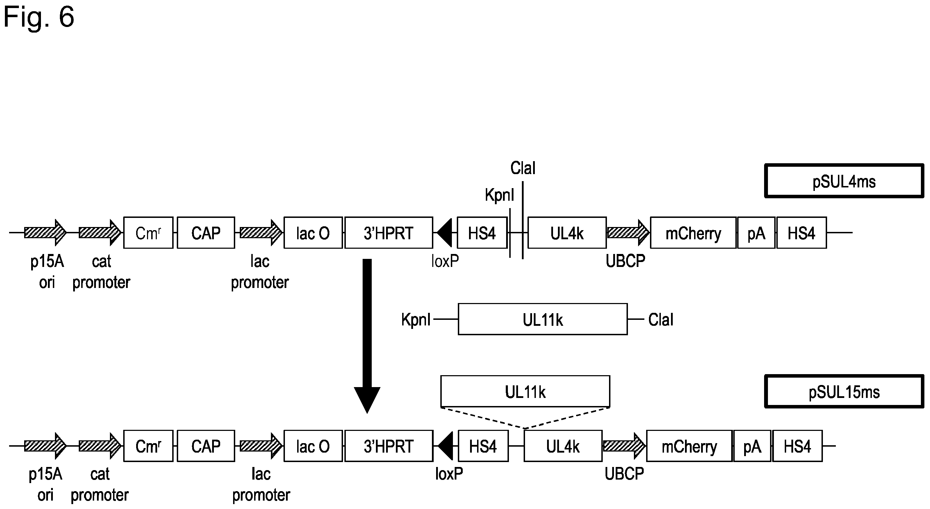

[Figure 6] Figure 6 is a diagram showing a scheme to construct pSUL15ms vector.

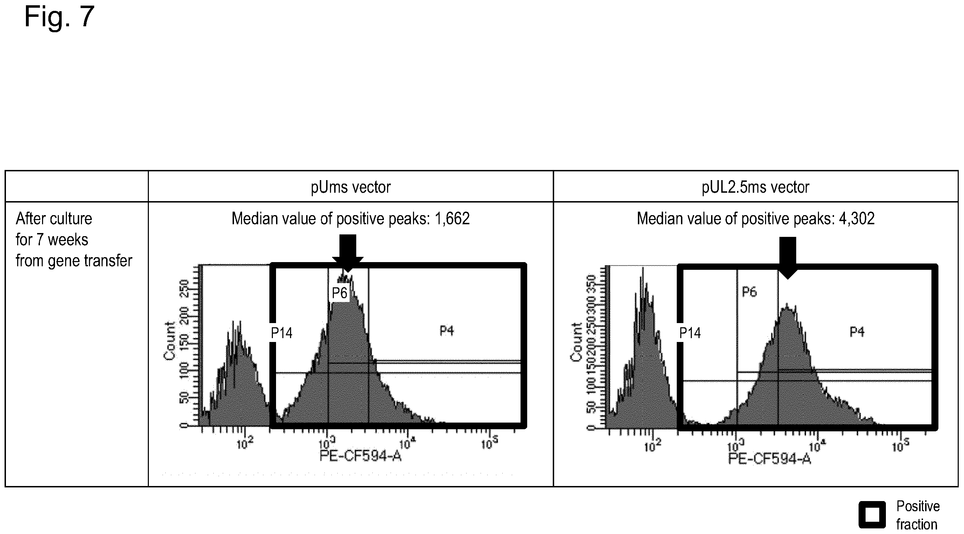

[Figure 7] Figure 7 is a diagram in which results of conducting FACS analysis with the expression of a red fluorescent protein as an index after culture for 7 weeks from gene transfer were compared as to CHO cells (HAT-resistant clones) harboring 21HAC vector having an insert of pUms vector or pUL2.5ms vector. Each portion enclosed by the thick-line box depicts a positive fraction. The left graph shows data on FACS analysis as to the CHO cells having the pUms vector, and a median value of positive peaks is 1662. The right graph shows data on FACS analysis as to the CHO cells having the pUms2.5ms vector, and a median value of positive peaks is 4302.

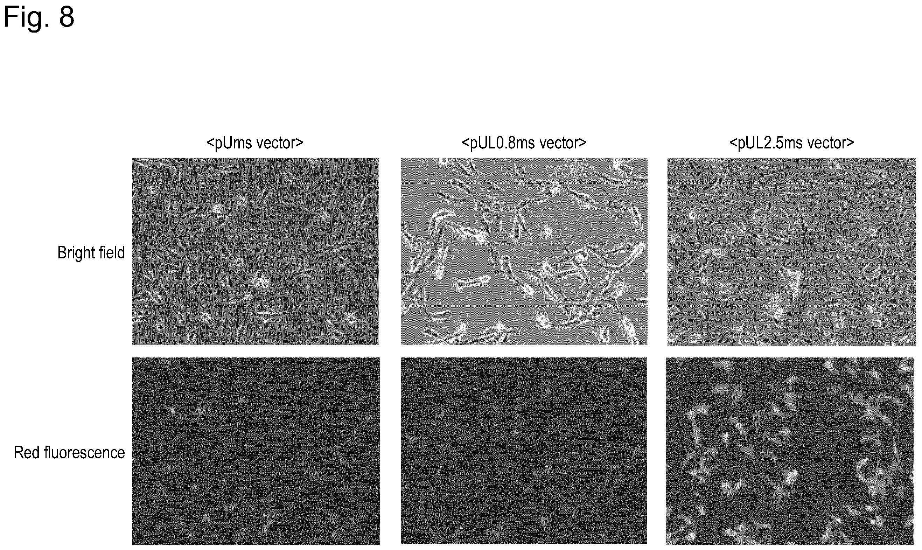

[Figure 8] Figure 8 is photographs showing results of observing the expression of a red fluorescent protein after 2 weeks from transfer as to MSC cells harboring pUms vector, pUL0.8ms vector, or pUL2.5ms vector. In Figure 8, the upper photographs are bright field images, and the lower photographs are red fluorescence images. In Figure 8, the left photographs show the cells harboring the pUms vector, the central photographs show the MSC cells harboring the pUL0.8ms vector, and the right photographs show the cells harboring the pUL2.5ms vector.

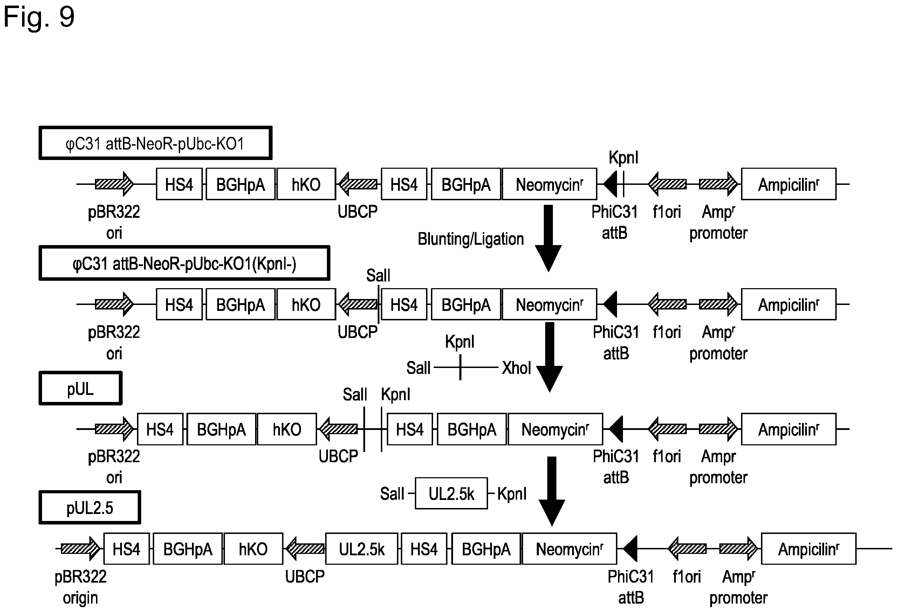

[Figure 9] Figure 9 is a diagram showing a scheme to construct an orange expression vector pUbC-hKO+MCS vector (pUL vector) for human artificial chromosome transfer and pUL2.5 vector with a genomic sequence of approximately 2.5 kb at 5' upstream of human UbC gene added to the pUL vector.

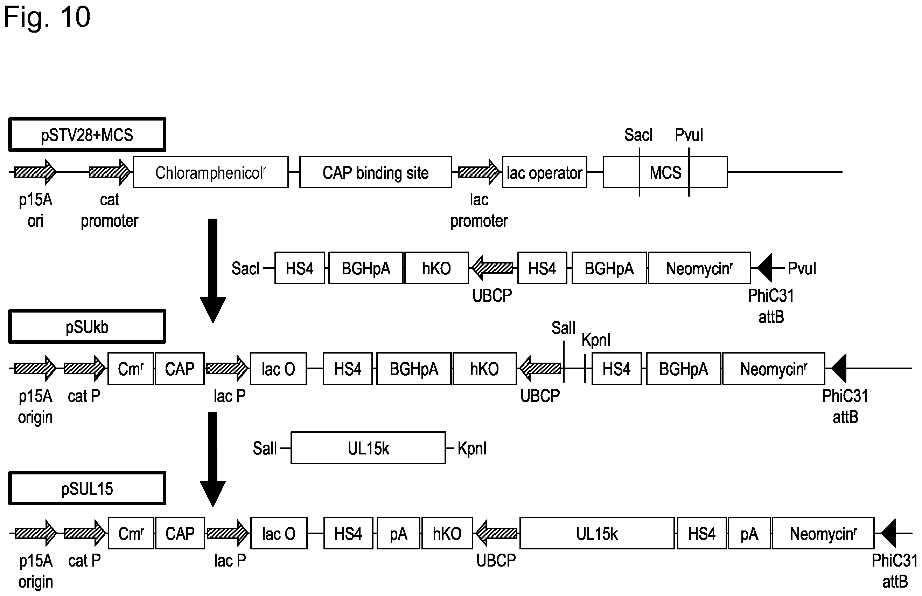

[Figure 10] Figure 10 is a diagram showing a scheme to construct pSUL15 vector with a genomic sequence of approximately 15 kb at 5' upstream of human UbC gene added to an orange expression vector pSUkb vector for human artificial chromosome transfer.

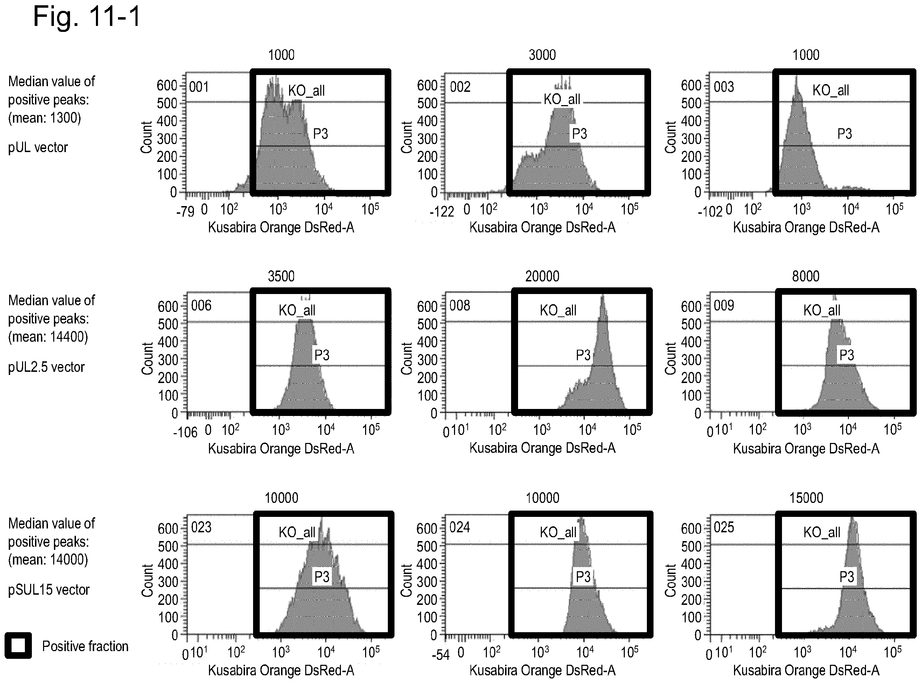

[Figure 11-1] Figure 11-1 is a diagram in which results of conducting FACS analysis with the expression of an orange fluorescent protein as an index were compared as to CHO cells (HAT-resistant clones: 6 clones each) harboring Basal-HAC vector having an insert of pUL vector, pUL2.5 vector or pSUL15 vector. Each portion enclosed by the thick-line box depicts a positive fraction. The upper graphs show data on FACS analysis as to the CHO cells having the pUL vector, and a median value of positive peaks is 1300. The middle graphs show data on FACS analysis as to the CHO cells having the pUL2.5 vector, and a median value of positive peaks is 14400. The lower graphs show data on FACS analysis as to the CHO cells having the pSUL15 vector, and a median value of positive peaks is 14000.

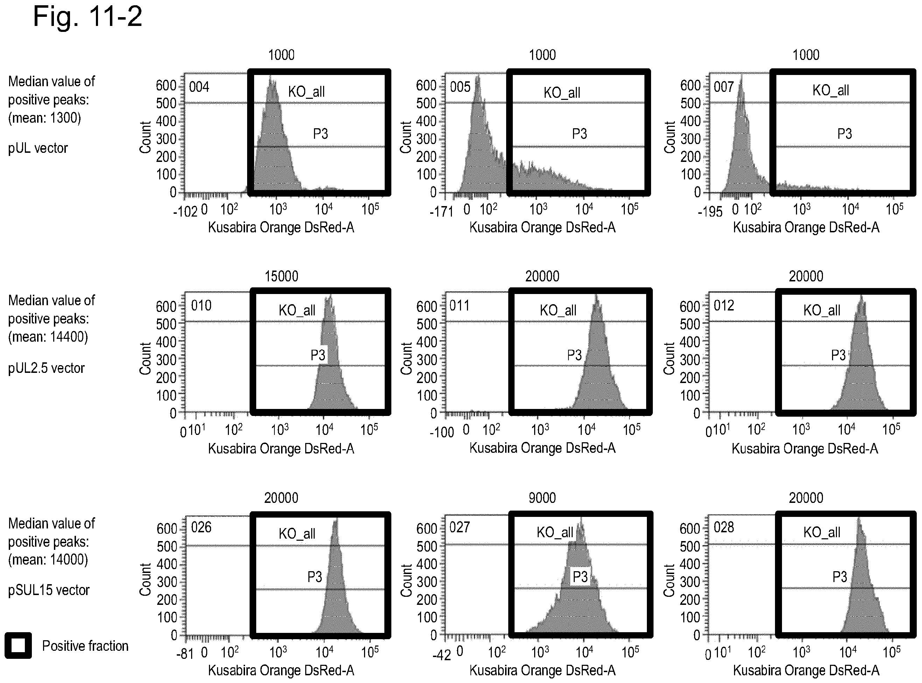

[Figure 11-2] Figure 11-2 is a diagram in which results of conducting FACS analysis with the expression of an orange fluorescent protein as an index were compared as to CHO cells (HAT-resistant clones: 6 clones each) harboring Basal-HAC vector having an insert of pUL vector, pUL2.5 vector or pSUL15 vector. Each portion enclosed by the thick-line box depicts a positive fraction. The upper graphs show data on FACS analysis as to the CHO cells having the pUL vector, and a median value of positive peaks is 1300. The middle graphs show data on FACS analysis as to the CHO cells having the pUL2.5 vector, and a median value of positive peaks is 14400. The lower graphs show data on FACS analysis as to the CHO cells having the pSUL15 vector, and a median value of positive peaks is 14000.

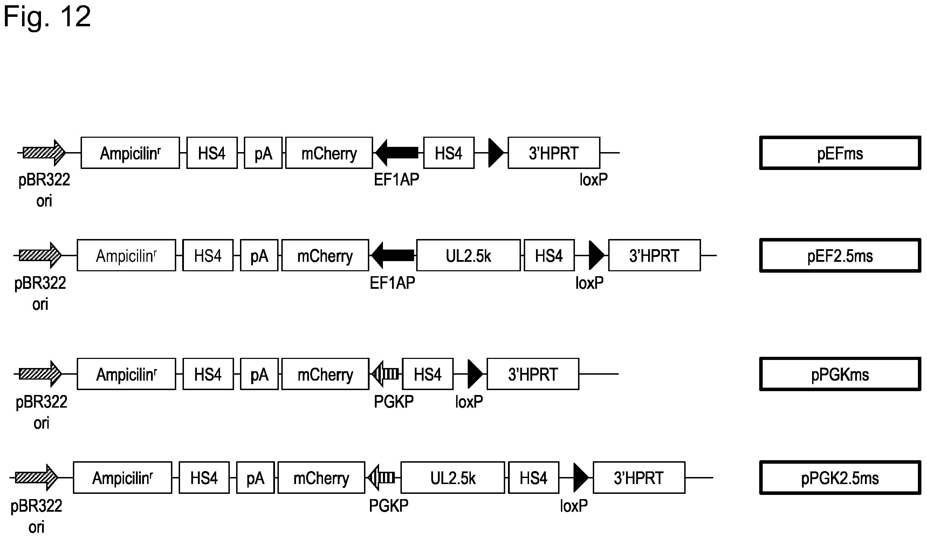

[Figure 12] Figure 12 is a diagram showing the structures of a mCherry expression vector pEFms vector using EF1α gene promoter, pEF2.5ms vector with a genomic sequence of 2.5 kb upstream of UbC gene added to the pEFms vector, a mCherry expression vector pPGKms vector using PGK gene promoter, and pPGK2.5ms vector with a genomic sequence of 2.5 kb upstream of UbC gene added to the pPGKms vector.

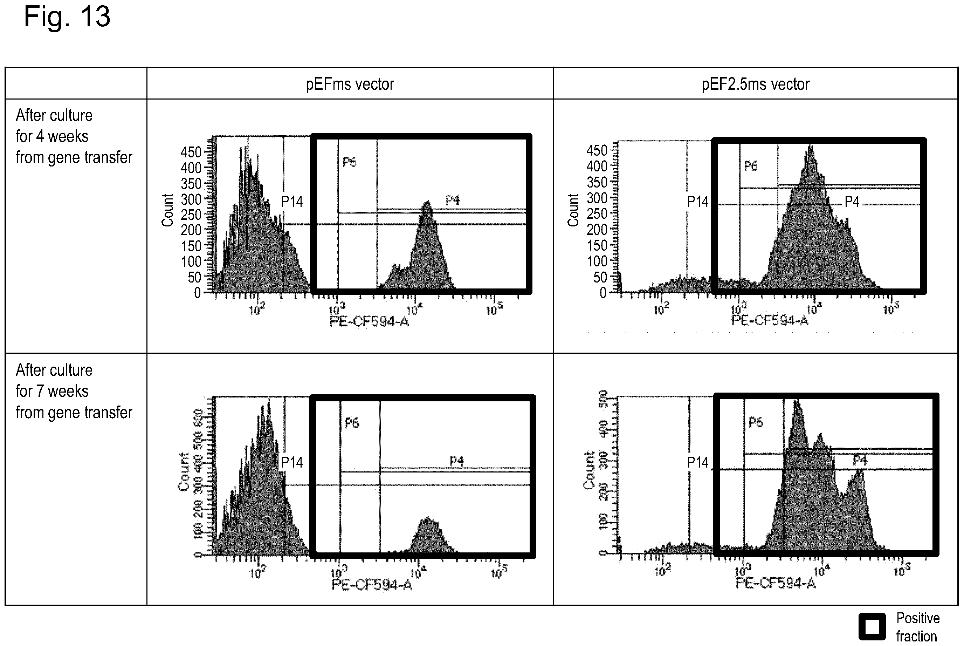

[Figure 13] Figure 13 is a diagram in which results of conducting FACS analysis with the expression of a red fluorescent protein as an index after culture for 4 weeks and culture for 7 weeks from transfer were compared as to CHO cells (HAT-resistant cell populations) harboring HAC vector having an insert of pEFms vector or pEF2.5ms vector. Each portion enclosed by the thick-line box depicts a positive fraction. The left graphs show data as to the CHO cells having the pEFms vector. The right graphs show data as to the CHO cells having the pEF2.5ms vector. The upper graphs show data obtained after the culture for 4 weeks from gene transfer, and the lower graphs show data obtained after the culture for 7 weeks from gene transfer.

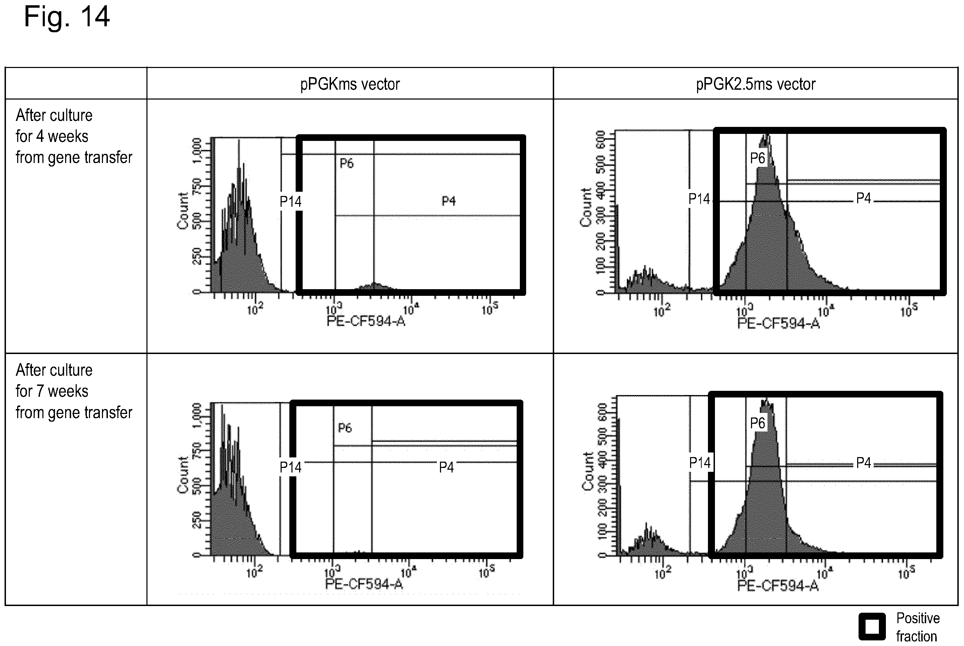

[Figure 14] Figure 14 is a diagram in which results of conducting FACS analysis with the expression of a red fluorescent protein as an index after culture for 4 weeks and culture for 7 weeks from transfer were compared as to CHO cells (HAT-resistant cell populations) harboring HAC vector having an insert of pPGKms vector or pPGK2.5ms vector. Each portion enclosed by the thick-line box depicts a positive fraction. The left graphs show data as to the CHO cells having the pPGKms vector. The right graphs show data as to the CHO cells having the pPGK2.5ms vector. The upper graphs show data obtained after the culture for 4 weeks from gene transfer, and the lower graphs show data obtained after the culture for 7 weeks from gene transfer.

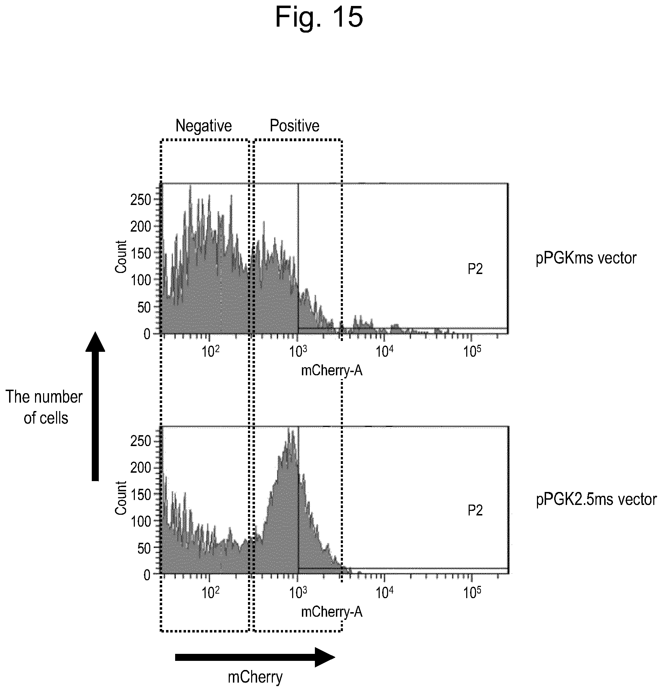

[Figure 15] Figure 15 is a diagram in which results of conducting FACS analysis with the expression of a red fluorescent protein as an index after culture for 4 weeks from transfer by electrical pulse were compared as to human mesenchymal stem cells (HAT-resistant cell populations) harboring HAC vector having an insert of pPGKms vector or pPGK2.5ms vector. The upper graph shows data as to the human mesenchymal stem cells having the pPGKms vector. The lower graph shows data as to the human mesenchymal stem cells having the pPGK2.5ms vector.

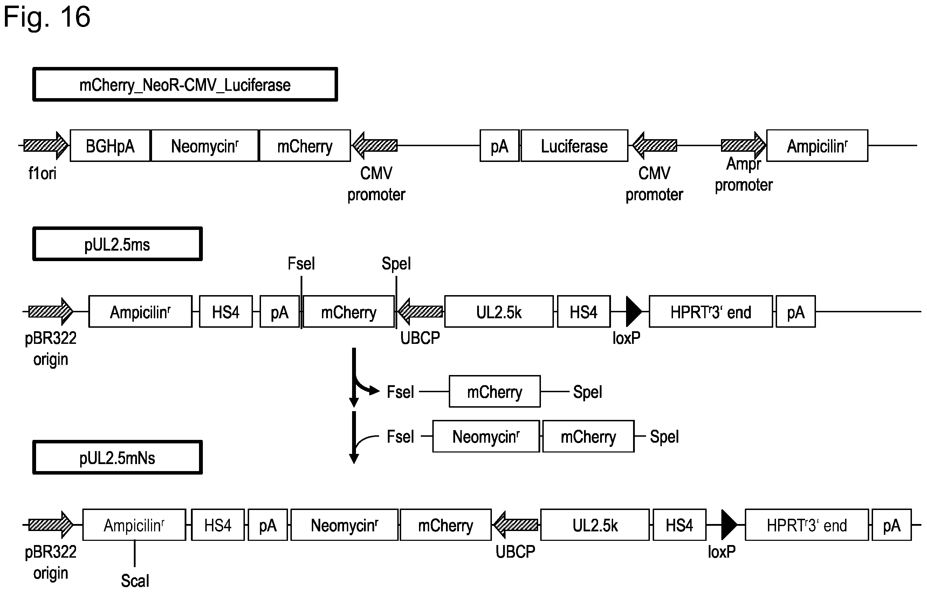

[Figure 16] Figure 16 is a diagram showing the structure of pUL2.5mNs vector obtained by inserting a mCherry-NeoR amplified fragment digested with SpeI and FseI to pUL2.5ms vector digested with SpeI and FseI.

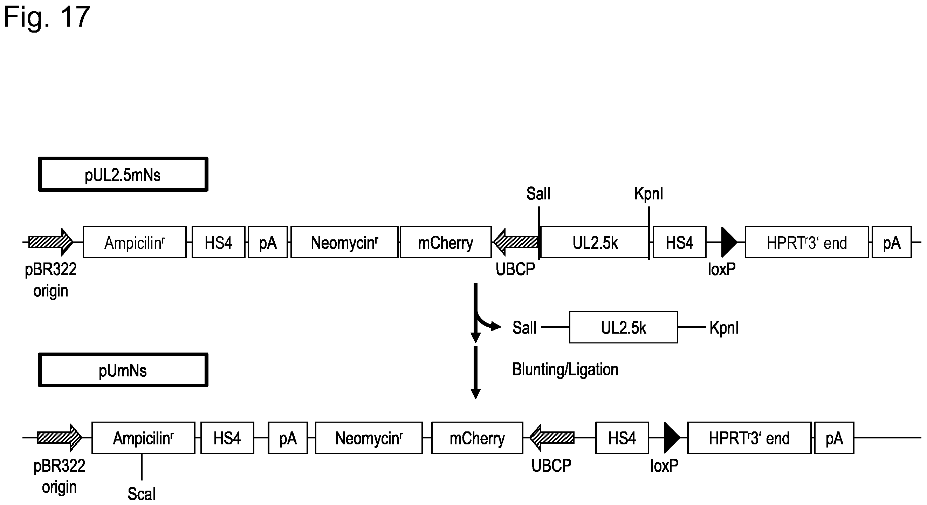

[Figure 17] Figure 17 is a diagram showing the structure of pUmNs vector obtained by deleting a region of 2.5 kb from pUL2.5mNs vector.

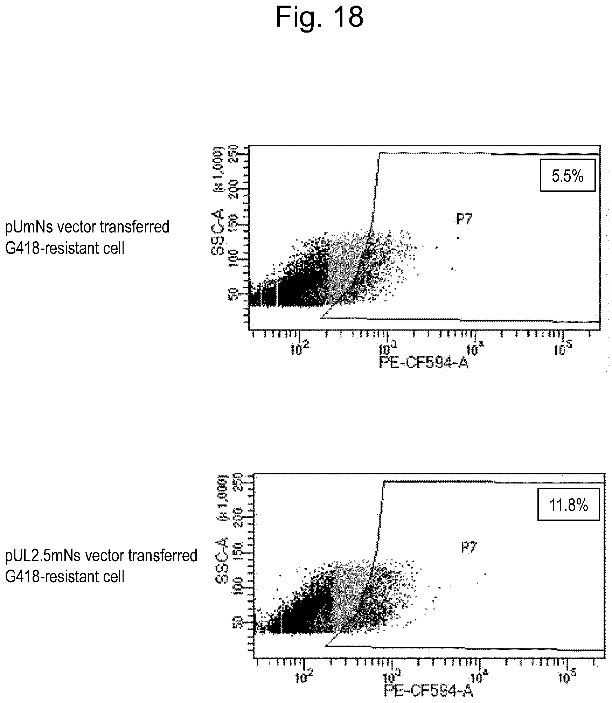

[Figure 18] Figure 18 is a diagram in which results of conducting FACS analysis with the expression of a red fluorescent protein as an index after culture for 2 weeks from transfer by electrical pulse were compared as to neomycin-resistant CHO cells (G418-resistant cells) harboring HAC vector having an insert of pUmNs vector or pUmL2.5ms vector. The upper graph shows data as to the G418-resistant cells having the pUmNs vector. The lower graph shows data as to the human mesenchymal stem cells having the pUL2.5mNs vector. In the G418-resistant cells having the pUmNs vector, the number of mCherry-positive (fraction P7) cells based on the total number of cells was 1136 (5.5%) out of 20826 cells. In the G418-resistant cells having the pUL2.5mNs vector, the number of mCherry-positive (fraction P7) cells based on the total number of cells was 1481 (11.8%) out of 12595 cells.

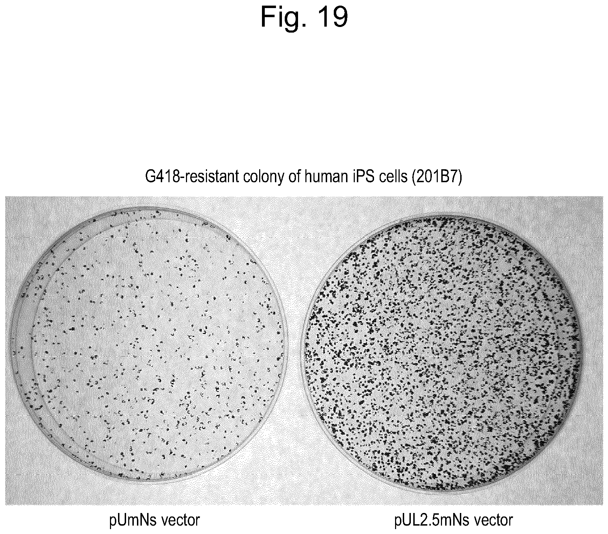

[Figure 19] Figure 19 is a photograph of G418-resistant colonies of human iPS cells (201B7) observed 7 days after transfer of pUmNs vector or pUmL2.5ms vector. As a result of performing vector transfer under the same conditions, a larger number of G418-resistant colonies appeared by the transfer of the pUmL2.5ms vector.

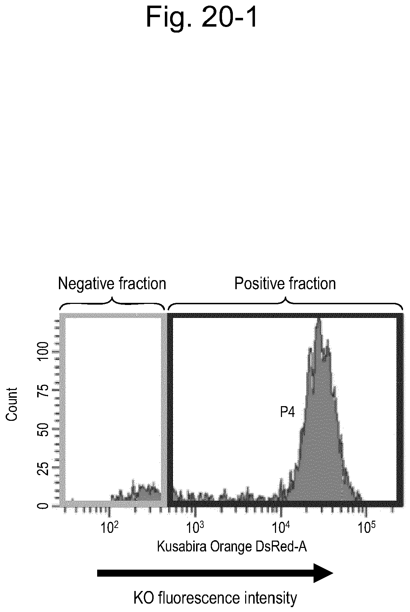

[Figure 20-1] Figure 20-1 shows results of conducting FACS analysis with the expression of an orange fluorescent protein as an index as to human iPS clones harboring Basal-HAC vector having an insert of pUL vector or pUL2.5 vector. Figure 20-1 shows data on FACS analysis of representative clones. A portion enclosed by the thick-line box depicts a Kusabira Orange (KO)-positive fraction. The abscissa depicts KO fluorescence intensity, and the ordinate depicts a count.

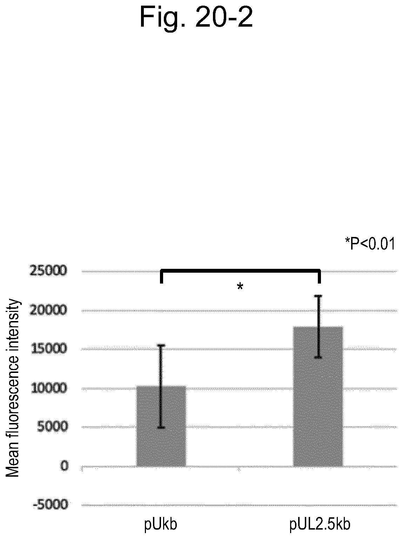

[Figure 20-2] Figure 20-2 is a diagram in which results of conducting FACS analysis with the expression of an orange fluorescent protein as an index were compared as to human iPS clones (HAT-resistant clones: 12 clones each) harboring Basal-HAC vector having an insert of pUL vector or pUL2.5 vector. Figure 20-2 shows mean fluorescence intensity from the iPS clones harboring KO. In Figure 20-2, the left bar depicts mean fluorescence intensity from the human iPS clones harboring the pUL vector, and the right value depicts mean fluorescence intensity from the human iPS clones harboring the pUL2.5 vector. *P < 0.01 represents that a significant difference was obtained at a significance level of 1% as a result of a t test.

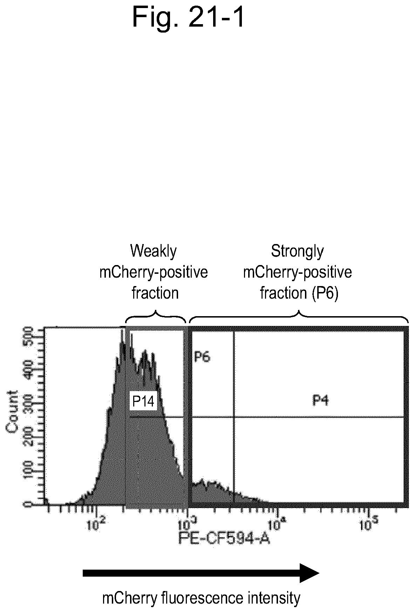

[Figure 21-1] Figure 21-1 shows results of evaluating the mCherry expression of CHO cell mixes (n = 3) harboring a mCherry-Neo expression vector. Neomycin-resistant CHO cell (G418-resistant cell) populations harboring HAC vector having an insert of pUmNs vector or pUmL2.5ms vector were subjected to FACS analysis on 2 weeks, 4 weeks, or 7 weeks from transfer by electrical pulse and evaluated for the ratio of strongly mCherry-positive cells to the total number of cells. In Figure 21-1, a portion enclosed by the thick-line box depicts a mCherry-Neo-positive fraction (P6). The abscissa depicts mCherry fluorescence intensity, and the ordinate depicts a count.

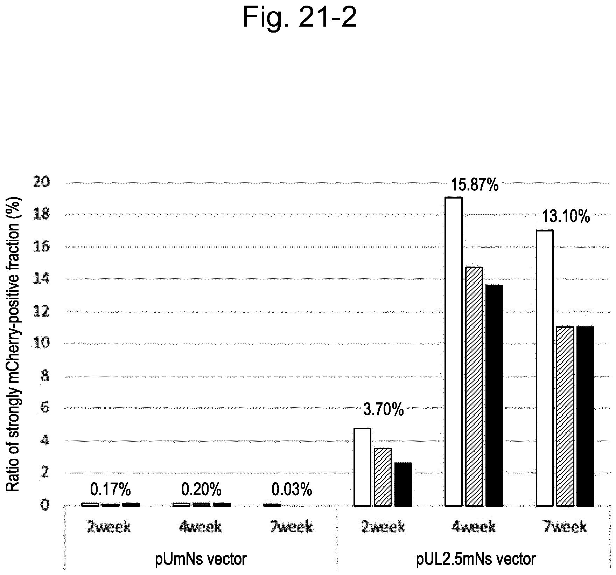

[Figure 21-2] Figure 21-2 shows results of evaluating the mCherry expression of CHO cell mixes (n = 3) harboring a mCherry-Neo expression vector. Neomycin-resistant CHO cell (G418-resistant cell) populations harboring HAC vector having an insert of pUmNs vector or pUmL2.5ms vector were subjected to FACS analysis on 2 weeks, 4 weeks, or 7 weeks from transfer by electrical pulse and evaluated for the ratio of strongly mCherry-positive cells to the total number of cells. Figure 21-2 is a diagram showing results of measuring the ratio of the number of cells in the strongly mCherry-positive fraction (P6 of Figure 21-1) to the total number of cells on 2 weeks (2week), 4 weeks (4week), or 7 weeks (7week) from transfer. The ratio (%) of the number of cells in the strongly mCherry-positive fraction in CHO cell populations having a random insert of mCherry (three independent experiments; n = 3) was compared between the pUmNs vector (three left bars: 2week, 4week, and 7week) and the pU2.5mNs vector (three right bars: 2week, 4 week, and 7 week).

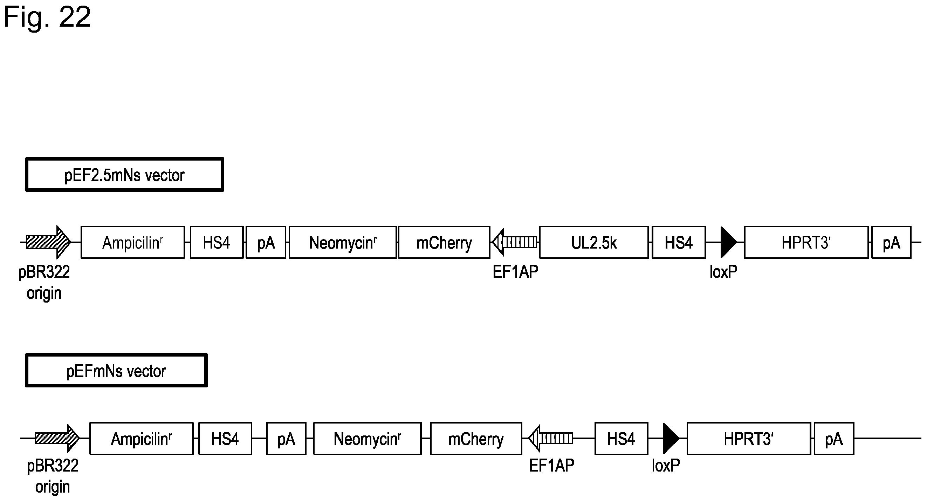

[Figure 22] Figure 22 is a diagram showing the structures of pEFmNs vector and pEF2.5mNs vector constructed by substituting mCherry gene in the pEFms vector and the pEF2.5ms vector (Figure 12) by mCherry-NeoR (neomycin-resistant) fusion gene.

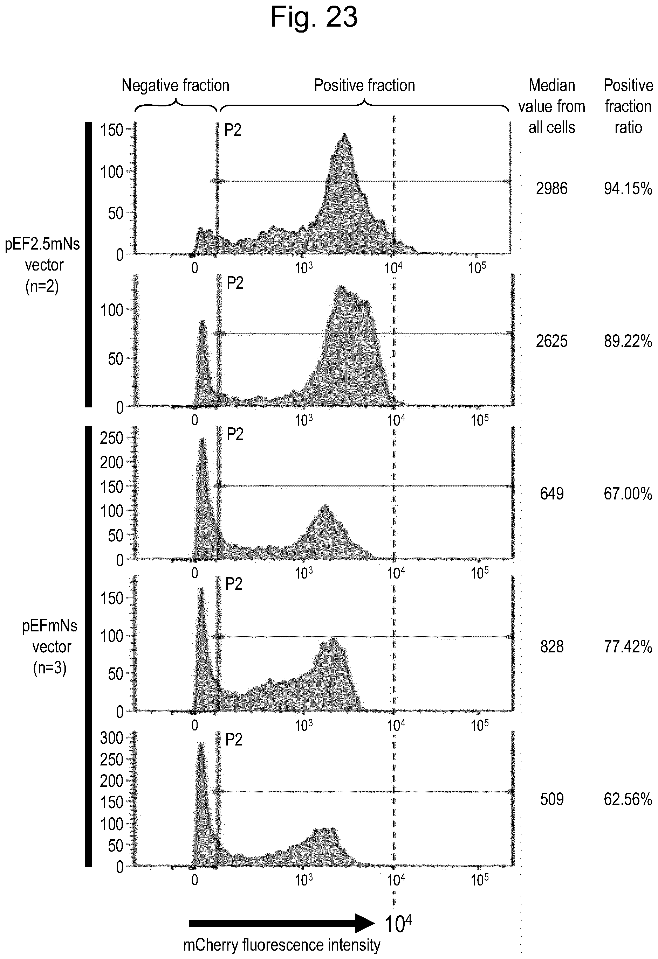

[Figure 23] Figure 23 shows results of studying an anti-silencing effect and an activity enhancing effect of an additional sequence element on EF1A-mCherry-NeoR expression unit in gene transfer by random insertion to CHO cells. Results of conducting FACS analysis with the expression of a red fluorescent protein as an index after culture for 4 weeks from transfer by electrical pulse were compared as to neomycin-resistant CHO cell populations harboring HAC vector having an insert of pEFmNs vector or pEFmNsms vector. The first and second graphs from above depict data on FACS analysis of the CHO cells having the pEF2.5mNs vector, and the third to fifth graphs from above depict data on FACS analysis of the CHO cells having the pEFmNs vector. Each data on FACS analysis also shows median mCherry fluorescence intensity from the total number of cells and the ratio of mCherry-positive cells thereto.

### Description of Embodiments

### 1. Promoter activation sequence of the present invention

The promoter activation sequence of the present invention comprises a nucleotide sequence represented by SEQ ID NO: 1, or a nucleotide sequence of 850 or more in a nucleotide sequence having a mutation in the nucleotide sequence. The nucleotide sequence represented by SEQ ID NO: 1 is a sequence positioned further upstream of an expression promoter of UbC gene on human chromosomal DNA. More specifically, the nucleotide sequence represented by SEQ ID NO: 1 is a nucleotide sequence of 2549 bp corresponding to positions from 124,927,045 to 124,929,593 of human chromosome 12. The nucleotide sequence of human chromosome 12 is disclosed under NC_000012.12 in GenBank of the National Center for Biotechnology Information (NCBI). The nucleotide sequence of SEQ ID NO: 1 is shown in Table 1.

**[Table 1]**

| SEQ ID NO: 1 |
|---|
| ccaggaagtt caggaaattg gctgacccag tgcatccatt tggaagtgta gtgggcatct 60 cgaaacgtaa catgcctgtg actgaagtct tgatttcctt actcactctg aggcacctgc 120 cctcgggcaa gagcatcacc atccgtccag tagcccagac agccgctggg tgtcatcctt 180 cacccccctt tctttcccct ttccctatat gaaatccatc aacaagtcct cccaccctcc 240 ttctgaagcc agtctcaaat tcgtcccttc atcaacacct ccaactgcct cctcgctgga 300 cttcctgctg ccactctggc tggccagtcc attctccacc cagcagccgg ggtgactttg 360 cagatggaaa tcatctcact cccccatggc tttccaagat ccagggcacc caggctcatc 420 tcctaggcct ccaaagatgg cgagatctgg tctccgcccc acattcagac cttgccccct 480 cccctcactc ctcttcccct ccacctcttc catcatgact tctttctgtc cctctgacaa 540 gccaacctgg tttcaacctc aggacctttg tactagctgt tccctccgtc tgtgatgctc 600 ttcctccagg tcttcccgtg gctcttcagt cttctggtct tctcagatct tcccttctct 660 tgctgccccc acagtgcagg tattttattt ttcttagcat ttactattat gtggacttat 720 tttgttattg tatttggctt tttttttttt ttttaataag atagggagct cactattttg 780 cccagactgg tctcgaactc ctacccacaa gcgagtctgc tttcacctgg gcctcccaaa 840 gctctgggat tactaggttg agcccccgca cctggcctgt taattgtatt tgttgatggg 900 tcagacgatt attaggtgct cggcgaatgc ttcttgaatg acttcaggtc atgagggatt 960 tcctgattct ttcccggaca acacccgcac cgtccaaccg cccacttccc gaagcgaaga 1020 gcccagtctg gatgccaacc tgttgacgtc ttcccgcttg gaagctgagc aggtgctcac 1080 aagcatggag gccacactcg gaggccggat ctgagtcact ccctgcccga ggaacgctgc 1140 gttccggctg acgcgtcctc ccgtaggccc tggcgaggtc aacatccctg ccacgcctcg 1200 cgctgcttgc ggagctgcgc ccaggggatg acctttttcc tttgcgcgga gaagggggct 1260 tgagaaaccc gagctctgaa ccaggtggga ttcattcatt cacgcatgca ttcattcatg 1320 tagtcagtca gtcaatcagt caacaaatat ttgtcaaggc cccacaatgt gctaggcact 1380 gttctaggta ctgagggaca cagcggtgcc cgaggtagac aaggttcctg ctcctcttgt 1440 gtttgtcatg cttgctgggg agacggacaa ggaaaatgat aaagttaaca gtaaatagta 1500 aaggctgggc catggcggga gatgtgatca gagaaggctt ctcagaggtc gtggccttga 1560 cagacgaaca ggccaccgca cgcagtccct gctgaacctg cattcccagc gtctgtgtca 1620 aagacccggg gcggcaaaac gaccaggagg ataaaagagc atcgcagtcg taacggctgc 1680 cgaaatagct cagttgggag agcgttagac tgaagatcta aaggtccctg gttcgatccc 1740 gggtttcggc acttcctctt tttccttccc cccgggggaa aaaggaggtg catagacgaa 1800 gccgattcct tccaattgat caaaggcttt tactttctat atttaacaaa gccccgagca 1860 gtgtccgcga ggagccagag ggaaaggaaa ctccccctcc actccccgtt tgggtaaatc 1920 aaataaggtc aagtttttcc cgtctccacc tcctccatgt ctcaaatgca catttgttga 1980 tgcctgtttg ttcatttttg ctttctgttt tttaacgtct gctttctgaa ctggaaacga 2040 tcgccttaaa atggcatgaa gtcaagtcgg acctgttgtt ttgtgggaaa tacaagatac 2100 ttaattcttt ggaaagtgtt tagggaaagc tacgtttacc ttaaaacagt ctcttgaaga 2160 acctgtttta tgggcggtcc tcgttagtat agtggtgagt atccccgcct gtcacgcggg 2220 agaccggggt tcgattcccc gacggggagg ccaagtacgt ttttaccatt cttccgtatt 2280 tttccttact cttcctatga agaataaacc cggcaggtaa acgggactct ggttctgtgg 2340 ttgtctctgg cctcgttgcg tgccacactg ttacaggaaa gcaagaaaaa ggcagtgctc 2400 gcatgtggaa gtgttcatcc atttcgttct ttttttttgg agaccatttc tttttttttt 2460 tttttttcct cagacagagt ctcgctctgt ctcccaggct ggagtgcagt ggcccgatct 2520 cagctcactg taacctccgc cttccgggt 2549 |

The promoter activation sequence of the present invention most preferably consists of the whole nucleotide sequence of SEQ ID NO: 1, i.e., the nucleotide sequence of 2549 bp of SEQ ID NO: 1. However, the promoter activation sequence of the present invention may be a shorter partial sequence of SEQ ID NO: 1. The promoter activation sequence of the present invention may consist of, for example, 1500 bp or more, 1000 bp or more, or 850 bp or more of the nucleotide sequence of SEQ ID NO: 1.

As mentioned above, the promoter activation sequence of the present invention also encompasses a nucleotide sequence having a mutation in the nucleotide sequence of SEQ ID NO: 1. Specifically, examples of the nucleotide sequence having a mutation in the nucleotide sequence of SEQ ID NO: 1 can include nucleotide sequences having preferably 85% or higher, more preferably 90% or higher, more preferably 95% or higher, more preferably 97% or higher, further preferably 98% or higher, particularly preferably 99% or higher homology to the nucleotide sequence represented by SEQ ID NO: 1. The homology between the nucleotide sequences can be determined using algorithm BLAST by Karlin and Altschul [Pro. Natl. Acad. Sci. USA, 90, 5873 (1993)] or FASTA [Methods Enzymol., 183, 63 (1990)]. In the case of analyzing the nucleotide sequences by BLASTN based on BLAST, parameters can be set to, for example, score = 100 and wordlength = 12.

The promoter activation sequence of the present invention also encompasses a nucleotide sequence derived from the nucleotide sequence represented by SEQ ID NO: 1 by the deletion, substitution, addition, or insertion of one to several bases. In this context, the term "several" means 10 or less, preferably 8 or less, more preferably 6 or less, most preferably 4 or less.

The promoter activation sequence of the present invention also encompasses a nucleotide sequence that hybridizes under stringent conditions to a complementary strand of the nucleotide sequence represented by SEQ ID NO: 1. In this context, examples of the stringent conditions include conditions described in Current Protocols in Molecular Biology, John Wiley & Sons, 6.3.1-6.3.6, 1999, for example, hybridization in 6 × SSC (sodium chloride/sodium citrate) at 45°C followed by washing once or more with 0.2 × SSC and 0.1% SDS at 50 to 65°C. Those skilled in the art can appropriately select hybridization conditions that provide stringency equivalent thereto.

As shown in Examples described below, an intended effect was not obtained when a nucleotide sequence of "approximately 0.8 kb" in Figure 1, a portion of the sequence of SEQ ID NO: 1, was used as the promoter activation sequence of the present invention. Accordingly, a nucleotide sequence of 850 or more in the nucleotide sequence represented by SEQ ID NO: 1 is considered to be necessary as the promoter activation sequence of the present invention. The nucleotide sequence of "approximately 0.8 kb" in Figure 1 is represented by SEQ ID NO: 2, and the nucleotide sequence is shown in Table 2.

**[Table 2]**

| SEQ ID NO: 2 |
|---|
| cgcacgcagt ccctgctgaa cctgcattcc cagcgtctgt gtcaaagacc cggggcggca 60 aaacgaccag gaggataaaa gagcatcgca gtcgtaacgg ctgccgaaat agctcagttg 120 ggagagcgtt agactgaaga tctaaaggtc cctggttcga tcccgggttt cggcacttcc 180 tctttttcct tccccccggg ggaaaaagga ggtgcataga cgaagccgat tccttccaat 240 tgatcaaagg cttttacttt ctatatttaa caaagccccg agcagtgtcc gcgaggagcc 300 agagggaaag gaaactcccc ctccactccc cgtttgggta aatcaaataa ggtcaagttt 360 ttcccgtctc cacctcctcc atgtctcaaa tgcacatttg ttgatgcctg tttgttcatt 420 tttgctttct gttttttaac gtctgctttc tgaactggaa acgatcgcct taaaatggca 480 tgaagtcaag tcggacctgt tgttttgtgg gaaatacaag atacttaatt ctttggaaag 540 tgtttaggga aagctacgtt taccttaaaa cagtctcttg aagaacctgt tttatgggcg 600 gtcctcgtta gtatagtggt gagtatcccc gcctgtcacg cgggagaccg gggttcgatt 660 ccccgacggg gaggccaagt acgtttttac cattcttccg tatttttcct tactcttcct 720 atgaagaata aacccggcag gtaaacggga ctctggttct gtggttgtct ctgg 774 |

As already mentioned, Patent Literature 1 and Non Patent Literature 1 disclose that a DNA sequence of approximately 15 kb positioned upstream of a promoter of UbC gene on human chromosomal DNA enhances gene expression. Figure 1 shows a gene map of a region from 124,910,000 to 124,955,000 of human chromosome 12 obtained from the USCC genome browser.

In Figure 1, the left-pointing arrow depict regions containing UbC gene (left) and DHX37 gene (right) in chromosome 12. The bar line indicated by the term "Sequence of present application (approximately 2.5 bp)" below the line representing nucleotide numbers in human chromosome 12 depicts a region corresponding to the nucleotide sequence represented by SEQ ID NO: 1 (2549 bp). The left bar line indicated by the term "15-kb region" therebelow depicts a region corresponding to the nucleotide sequence of 15 kb disclosed in Patent Literature 1 and Non Patent Literature 1. This nucleotide sequence of 15 kb corresponds to a region from 124914981 to 124929646 of human chromosome 12 (NC_000012.12 Homo sapiens chromosome 12, GRCh38.p13 Primary Assembly).

Patent Literature 1 and Non Patent Literature 1 disclose that an activity enhancing effect was reduced as the promoter activation sequence was shortened to 15 kb, 4 kb, and 1.3 kb. Specifically, Patent Literature 1 and Non Patent Literature 1 disclose that activity enhancing effects of 4.5 times, 2.5 times, and 1.2 times were obtained with the amount of erythropoietin produced from erythropoietin gene serving as a gene of interest as an index in CHO cells harboring vectors with promoter activation sequences of 15 kb, 4 kb, and 1.3 kb linked to a promoter, respectively. According to the disclosure of Patent Literature 1 and Non Patent Literature 1, the long sequence (15 kb) had a higher activity enhancing effect, and the shorter partial sequences each used alone cannot achieve an activity enhancing effect equivalent to that of the sequence of 15 kb.

Accordingly, it has not been predicted that the promoter activation sequence of the present invention comprising the sequence of approximately 2.5 kb of the present invention (SEQ ID NO: 1), which is a partial sequence of the 15 kb described above, has a promoter activation effect at the same level as that of the sequence of 15 kb in the conventional technique.

In addition, it is generally considered that: all of general used EF1α, PGK, or UbC promoters are positioned at a region of 2 kb or less; and sequence elements necessary for functions of the housekeeping gene promoters are contained at a region of 1 to 2 kb containing a transcriptional initiation site. Accordingly, in this respect as well, it has not been predicted that the nucleotide sequence of SEQ ID NO: 1 of the present invention (2549 bp) which is distant by approximately 13 kb from human UbC gene promoter and is positioned near the middle between the promoter and adjacent DEAH-box helicase 37 (DHX37) gene has an activity enhancing effect equivalent to that of the region of approximately 15 kb.

For the reasons mentioned above, it was not expected that the nucleotide sequence consisting of SEQ ID NO: 1 or a partial sequence thereof (i.e., the promoter activation sequence of the present invention) had a function as the promoter activation sequence, and the effect is surprising.

### 2. Expression vector of present invention

The expression vector of the present invention comprises the promoter activation sequence described in 1., a promoter, and a DNA of interest.

The DNA of interest according to the present invention is a DNA intended to obtain a protein encoded thereby through expression or a DNA that achieves an intended effect of gene therapy by transfer thereof, and includes, for example, a gene or gene locus, or a nucleic acid such as cDNA, recombinant DNA, or modified DNA.

The DNA of interest according to the present invention encompasses, for example, but not limited to, disease causative genes, therapeutic genes, chromosome fragments containing genes thereof, and genes necessary for cell differentiation, and can include genes or DNA encoding proteins, for example, cytokines, interferon, interleukin, chemokines (factors having cell migratory activity), granulocyte-colony stimulating factors, tumor necrosis factors, growth factors (e.g., platelet-derived growth factors, vascular endothelial growth factors, hepatocyte growth factors, keratinocyte growth factors, nerve growth factors, epithelial stem cell growth factors, and hematopoietic stem cell growth factors), nutritional factors (e.g., neurotrophic factors and brain-derived neurotrophic factors), erythropoietin, blood-clotting proteins, platelet production promoters, hormones, antibodies (e.g., monoclonal antibodies and recombinant antibodies such as scFv), and enzymes. The DNA of interest can further include therapeutic genes or DNA related to diseases such as muscular dystrophy, hemophilia, neurodegenerative diseases, autoimmune diseases, allergic diseases, genetic diseases, and tumors, and immune system genes or DNA such as chimeric antigen receptor (CAR), T cell receptor (TCR), and human leukocyte antigen (HLA).

A protein encoded by the DNA of interest according to the present invention is expressed therefrom. In the present specification, the term "protein" includes a protein, a polypeptide, and a peptide unless otherwise specified, and may be any industrially useful protein and is preferably a human-derived protein. Examples of the protein include arbitrary proteins for use in the treatment or prevention of the diseases described above or the diagnosis of the diseases.

The expression vector of the present invention may comprise the promoter activation sequence of the present invention at 5' upstream or at 3' downstream of the promoter. More preferably, the expression vector of the present invention comprises the promoter activation sequence of the present invention at 5' upstream of the promoter.

The promoter comprised in the vector of the present invention is not particularly limited, and any promoter can be used as long as a function as the promoter can be exerted in mammalian cells. A constitutive promoter always causes expression of the DNA of interest in a given amount and is therefore advantageous for the object of the present invention. Accordingly, preferably, the promoter used in the present invention is a constitutive expression promoter known in the art to activate constitutive expression of the expression vector, preferably a promoter of a housekeeping gene.

Examples of such a constitutive promoter can include nonviral promoters such as human polypeptide elongation factor 1α gene promoter (EF1α promoter), phosphoglycerate kinase promoter (PGK promoter), and human ubiquitin C (UBC) promoter, and viral promoters such as CMV promoter, SV40 promoter, and CAG. The promoter used in the present invention is particularly preferably EF1α promoter, PGK promoter, or UBC promoter used in Examples described below.

The expression vector of the present invention can be prepared by inserting a DNA cassette comprising the promoter activation sequence of the present invention, a promoter, and a DNA of interest to an expression vector. In the present invention, the expression vector to which the promoter activation sequence, a promoter, and the DNA of interest are inserted is not particularly limited as long as the expression vector has the promoter at a position appropriate for expressing the DNA of interest inserted in the vector and allows for expression of a protein from the DNA of interest in mammalian cells. One example of the DNA cassette can include, but is not limited to, pUL2.5ms having ampicillin resistance gene, HS4 (insulator sequence mentioned below), a polyA sequence, a coding region sequence of red fluorescent protein (mCherry) gene, a UBCP promoter sequence, the promoter activation sequence of the present invention (2.5 kb), and further HS4 (see Figure 4 mentioned in Examples).

In the present invention, a mammalian artificial chromosome vector or a plasmid vector is preferably used, though the present invention is not limited thereby. However, a conventional vector system such as a virus, YAC, BAC, PAC, or a cosmid may be used in the present invention, though the present invention is not limited thereby.

In this context, examples of the mammalian artificial chromosome vector can include, but are not particularly limited to, artificial chromosome vectors of primates such as humans, monkeys, chimpanzees, and gorillas, rodents such as mice, rats, hamsters, and guinea pigs, ungulates such as bovines, sheep, and goats, dogs, cats, and the like. The mammalian artificial chromosome vector is particularly preferably a human artificial chromosome (HAC) vector, a mouse artificial chromosome (MAC) vector, or a rat artificial chromosome (RAC) vector. A human artificial chromosome vector is the most preferable vector. Specific examples of the human artificial chromosome vector can include 21HAC based on human chromosome 21, 14HAC based on human chromosome 14, and 2HAC based on human chromosome 2.

In the present specification, the "artificial chromosome vector" refers to an artificially prepared chromosome-derived vector comprising the centromere of a chromosome derived from the mammal, a fragment proximal to the centromere of a long arm and, if any, a short arm (from which genes and the like have been removed as much as possible), and natural or artificial telomere.

The mouse artificial chromosome vector is described in, for example, JP Patent Nos. 5557217 and 4997544, and the human artificial chromosome vector is described in, for example, JP Patent No. 4895100.

The human chromosome for use in the preparation of the human artificial chromosome vector may be any of human chromosomes 1 to 22, X, and Y and is preferably any of chromosomes 1 to 22. Alternatively, a human iPS cell-derived chromosome can be used as a human chromosome vector. For the preparation of the human artificial chromosome, see, for example, methods described in JP Patent Publication (Kokai) No. 2010-004887 A (2010) and WO2008/013067, or approaches described in the literatures regarding the preparation of the mouse artificial chromosome vector.

The mouse chromosome for use in the preparation of the mouse artificial chromosome vector may be any of mouse chromosomes 1 to 19, X, and Y and is preferably any of chromosomes 1 to 19. For example, in the case of an artificial chromosome vector derived from a mouse chromosome 11 fragment, the long arm fragment consists of, for example, but not limited to, a long arm fragment from which a region distal to AL671968, or BX572640 (positioned on the more centromere side than AL671968), CR954170 (positioned on the more centromere side than AL671968 and BX572640), or AL713875 (positioned on the more centromere side than AL671968) of the long arm of the chromosome 11 has been deleted. Alternatively, in the case of a mouse artificial chromosome derived from a mouse chromosome 15 fragment, the long arm fragment consists of, for example, a long arm fragment from which a region distal to a position such as AC121307 or AC161799 has been deleted. Alternatively, in the case of a mouse artificial chromosome derived from a mouse chromosome 16 fragment, the long arm fragment consists of, for example, a long arm fragment from which a region distal to a position such as AC127687 or AC140982 has been deleted.

The artificial chromosome vector described above can further comprise a DNA sequence insertion site such as loxP (Cre recombinase recognition site), FRT (Flp recombinase recognition site), φC31attB and φC31attP (φC31 recombinase recognition sites), R4attB and R4attP (R4 recombinase recognition sites), TP901-1attB and TP901-1attP (TP901-1 recombinase recognition sites), or Bxb1attB and Bxb 1 attP (Bxb1 recombinase recognition sites) for inserting a desired nucleic acid (e.g., a gene, a gene locus, or a chromosome fragment). The artificial chromosome vector described above can comprise a site for inserting a desired nucleic acid sequence. Therefore, the integration of a desired nucleic acid to this site enables the desired nucleic acid to be expressed when the artificial chromosome vector is transferred to arbitrary cells.

The object of the present invention can be achieved by transferring a DNA cassette comprising the promoter activation sequence of the present invention, a promoter, and a DNA of interest to an artificial chromosome without the use of a particular DNA sequence insertion site for inserting a desired nucleic acid sequence.

Examples 13 and 14 described below are examples in which neither site-directed recombinase nor a recognition site of the enzyme was used in inserting the DNA cassette to the artificial chromosome vector. In this case, the DNA cassette is randomly inserted to an arbitrary site other than centromere and telomere in the vector, or the genome of the cells.

Alternatively, the DNA cassette may be inserted either specifically for the genome of cells or randomly by transferring another vector such as a plasmid vector or a virus vector having an insert of the DNA cassette to the cells. The DNA cassette may be inserted either to a desired target site or randomly by use of, for example, a gene targeting method or a genome editing technique as a technique of inserting the DNA cassette to the genome.

The genome editing is a technique of performing genomic DNA editing or gene modification using, for example, an artificial cleavage enzyme such as TALEN (transcription activator-like effector nuclease) or ZFN (zinc finger nuclease), or a CRISPR-Cas system. Among these systems, a CRISPR/Cas9 system has been found from the adaptive immune system against a bacterial or archaebacterial virus or plasmid and is capable of relatively conveniently constructing a vector and modifying a plurality of genes at the same time (Jinek et al., Science, 17, 337 (6096): 816-821, 2012; and Sander et al., Nature Biotechnology, 32 (4): 347-355, 2014). This system comprises Cas9 nuclease which cleaves double-stranded DNA on the genome, and guide RNA (sgRNA or crRNA (CRISPR RNA) + tracrRNA (trans-activating CRISPR RNA)) having a sequence of approximately 20 base pairs that recognizes a target sequence on the genome. By their coexpression in cells, the gRNA recognizes a PAM sequence proximal to the target sequence and specifically binds to target genomic DNA while the Cas9 protein induces a double-stranded break (DSB) at 5' upstream of the PAM sequence (NGG (N = C, G, A, or T)). In this event, the DNA cassette can be inserted to the cleavage site provided that cells contains a vector comprising the DNA cassette.

Preferably, a reporter gene may be inserted, in addition to the sequence of the gene or locus of interest, to the mammalian artificial chromosome vector. Examples of reporter genes include, but are not particularly limited to, fluorescent protein genes (e.g., green fluorescent protein (GFP or EGFP) gene and yellow fluorescent protein (YFP) gene), tag-protein-encoding DNA, β-galactosidase gene, and luciferase gene.

The mammalian artificial chromosome vector may further comprise a selection marker gene. A selection marker is effective when selecting a cell transformed with the vector. As a selection marker gene, either or both of a positive selection marker gene and a negative selection marker gene are exemplified. Examples of positive selection marker genes include drug-resistant genes such as neomycin-resistant gene, ampicillin-resistant gene, blasticidin S (BS)-resistant gene, puromycin-resistant gene, geneticin (G418)-resistant gene, and hygromycin-resistant gene. In addition, examples of negative selection marker genes include herpes simplex thymidine kinase (HSV-TK) gene and diphtheria toxin A fragment (DT-A) gene. In general, HSV-TK is used in combination with ganciclovir or acyclovir.

The artificial chromosome vector serving as the expression vector according to the present invention comprises a nucleic acid insertion site to which a nucleic acid comprising the promoter activation sequence of the present invention, a promoter, and a DNA of interest can be inserted. The nucleic acid is inserted to this site.

At least one insulator sequence may be allowed to reside in the vicinity of the nucleic acid insertion site or on both sides of the insertion site in the artificial chromosome of the present invention. The insulator sequence has an enhancer blocking effect (i.e., neighboring genes are not influenced by each other) or a chromosome boundary effect (i.e., a region that assures gene expression and a region that suppresses gene expression are separated and distinguished from each other). Such a sequence may encompass, for example, human β globin HS1 to HS5 or chicken β globin HS4.

A nucleic acid comprising the promoter activation sequence of the present invention, a promoter, and a DNA of interest can be transferred to the donor cell, for example by using a gene transfer technique such as a genome editing method or a site-directed recombination method, onto an artificial chromosome such as a human artificial chromosome or a mammalian artificial chromosome in the donor cells. After transfer of the nucleic acid, the donor cells comprising the DNA of interest can be detected and recovered by, for example, a HAT selection method.

### 3. Mammalian cell of present invention

A mammalian cell comprising the expression vector of the present invention (hereinafter, simply referred to as the mammalian cell of the present invention) can be prepared by transferring the vector for foreign gene expression described above to a mammalian cell serving as a host cell.

The mammalian cell that is used as a host cell in the present invention is not particularly limited as long as the mammalian cell can cause nucleic acid amplification of the DNA of interest. Examples thereof include mammalian somatic cells, mammalian stem cells, established mammalian cell lines, human cells, and rodent cells (hamster cells, mouse cells, rat cells, etc.). As a preferred example, human cells or hamster cells are particularly preferably used.

Human-derived stem cells, for example, human pluripotent stem cells or human somatic stem cells, can be used as the human cells. Particularly preferably, human mesenchymal stem cells (MSC cells) are used as the human somatic stem cells, and human induced pluripotent stem cells (iPS cells) are used as the human pluripotent stem cells.

Mouse- or rat-derived stem cells, for example, mouse- or rat-derived pluripotent stem cells or somatic stem cells, can be used as the mouse cells or the rat cells. Mouse or rat MSC cells are particularly preferably used as the mouse or rat somatic stem cells. Mouse or rat induced pluripotent stem cells (iPS cells) are particularly preferably used as the mouse or rat pluripotent stem cells, and mouse or rat embryonic stem cells (ES cells) are also particularly preferably used. As with the case of mouse ES cells (M.J. Evans and M.H. Kaufman, Nature 1981; 292 (5819): 154-156), rat ES cells are pluripotent and self-reproducible cell lines that are established from the inner cell mass of the rat blastocyst stage embryo or 8-cell-stage embryo.

Chinese hamster ovary cells (CHO cells) are preferably used as the hamster cells. More specifically, for example, CHO-K1, CHO-S, or DG44 is particularly preferred.

Examples of the somatic cells can include, but are not limited to, hepatocytes, enterocytes, renal cells, splenocytes, lung cells, cardiac cells, skeletal muscle cells, brain cells, skin cells, bone marrow cells, fibroblasts, hematopoietic stem cells, T cells, and B cells.

In the present specification, the "embryonic stem cells" or the "ES cells" are pluripotent and semi-immortalized stem cells that are established from an inner cell mass of a blastocyst of a fertilized egg derived from a mammal (M. J. Evans and M. H. Kaufman (1981) Nature 292: 154-156; J. A. Thomson et al. (1999) Science 282: 1145-1147; J. A. Thomson et al. (1995) Proc. Natl. Acad. Sci. USA 92: 7844-7848; J. A. Thomson et al. (1996) Biol. Reprod. 55: 254-259; J. A. Thomson and V. S. Marshall (1998) Curr. Top. Dev. Biol. 38: 133-165).

Cells artificially induced by the reprogramming of somatic cells, which have properties equivalent to the ES cells, are "induced pluripotent stem cells" or "iPS cells" (K. Takahashi and S. Yamanaka (2006) Cell 126:663-676; K. Takahashi et al. (2007) Cell 131: 861-872; J.Yu et al. (2007) Science 318: 1917-1920). The iPS cells can form colonies in approximately 3 to 5 weeks by introducing certain reprogramming factors (DNA or proteins) into somatic cells (including somatic stem cells), and culturing and subculturing the cells in an appropriate medium. A known combination of the reprogramming factors is, for example, a combination of Oct3/4, Sox2, Klf4, and c-Myc; a combination of Oct3/4, Sox2, and Klf4; a combination of Oct4, Sox2, Nanog, and Lin28; or a combination of Oct3/4, Sox2, Klf4, c-Myc, Nanog, and Lin28 (K. Takahashi and S. Yamanaka, Cell 126: 663-676 (2006); WO2007/069666; M. Nakagawa et al., Nat. Biotechnol. 26: 101-106 (2008); K. Takahashi et al., Cell 131: 861-872 (2007); J. Yu et al., Science 318: 1917-1920 (2007); J. Liao et al., Cell Res. 18, 600-603 (2008)).

The mammalian artificial chromosome vector of the present invention can be introduced or transferred to arbitrary mammalian cells. Examples of the approach therefor include microcell fusion, lipofection, a calcium phosphate method, microinjection, and electroporation. The approach is preferably microcell-mediated chromosome transfer disclosed in WO2020/075823.

The microcell-mediated chromosome transfer is a method for transferring a mammalian artificial chromosome vector into a desirable cell by microcell fusion between a cell capable of forming microcells and comprising the mammalian artificial chromosome vector and the desirable cell. The cell capable of forming microcells is treated with a polyploid inducer (e.g., colcemid or colchicine) to form multinucleated micronucleate cells, which can then be treated with cytochalasin to form microcells, followed by cell fusion of the microcells to the desirable cell.

The mammalian cell comprising the expression vector of the present invention can achieve stable high expression of DNA intended to be expressed, owing to the presence of the promoter activation sequence of the present invention comprised in the expression vector. Examples mentioned below have demonstrated that the stable high expression of such DNA of interest is brought about by an expression enhancing activity and/or an anti-silencing activity against the DNA of interest.

### 4. Protein production method of present invention

A desired protein can be produced by culturing the mammalian cell described in 3. in a medium for animal cell culture, and collecting a protein encoded by the DNA of interest (hereinafter, referred to as the desired protein) from a culture product obtained by the culture. As mentioned above, the presence of the promoter activation sequence of the present invention brings about stable high expression of the DNA of interest.

The culture of the animal cell of the present invention can be performed in accordance with a usual method for use in mammalian cell culture.

The protein production method of the present invention includes a method of producing the protein within host cells, a method of secreting the protein outside of the host cells, or a method of producing the protein on outer membranes of host cells. An appropriate method can be selected by changing the host cells used or the structure of the protein to be produced.

A medium suitable for the type of the cell used is selected as the culture medium for the mammalian cell. For example, MEM medium, DMEM medium, RPMI medium, F12 medium, Macy's 5A medium, or a mixed medium thereof (e.g., DMEM/F12) can be used as a basal medium usually used. The basal medium can be further replenished with at least one component, for example, a supplement such as serum, a serum replacement, an antibiotic, glutamic acid, or pyruvic acid. The pH of the medium is, for example, 7.2 to 7.4.

Exemplary culture of iPS cells involves using a mitomycin C-treated mouse embryonic fibroblast cell line (e.g., STO) as feeder cells, and culturing somatic cells (e.g., approximately 10⁴ to 10⁵ cells/cm²) harboring the vector at a temperature of approximately 37°C using a medium for an ES cell on the feeder cell layer. The feeder cells are not always necessary (Takahashi, K. et al., Cell 131, 861-872, (2007)). Examples of the basic medium include Dulbecco's modified Eagle's medium (DMEM), Ham's F-12 medium, and mixed media thereof. For example, a medium for a mouse ES cell or a medium for a primate ES cell (Reprocell Inc.) can be used as the medium for an ES cell. For example, human iPS cells or human ES cells can be cultured under feeder-free conditions using StemFit AK02N medium (Takara Bio Inc.) free from an animal-derived component.

As for rat ES cells, for example, rat blastocysts with egg zona pellucida dissolved are cultured on mouse embryonic fibroblast (MEFF) feeder using a medium containing leukemia inhibitory factor (LIF), and outgrowth formed from the blastocysts is dispersed 7 to 10 days later, then transferred to MEF feeder, and cultured so that ES cells appear approximately 7 days later. The preparation of the rat ES cells is described in, for example, K. Kawaharada et al., World J. Stem Cells 2015; 7 (7): 1054-1063. Mouse ES cells can be cultured in the same manner as in the rat ES cells.

The culture is performed in an atmosphere of air containing approximately 2 to 10% (usually, approximately 5%) CO₂, and a temperature of approximately 35 to 40°C (usually, approximately 37°C) is suitable.

The culture method may be any of batch culture, flask culture, fed-batch culture, continuous culture, suspension culture, shake culture, stirring culture, circulation culture, and the like. In order to suppress cell aggregation, a method of allowing a culture solution to pass through a hollow fiber filter to disintegrate the aggregate during culture may be used.

An apparatus for use in animal cell culture can be used as a culture apparatus. The apparatus is preferably equipped with aeration, temperature control, stirring, pH adjustment, and DO adjustment functions. Culture can be performed using, for example, a fermenter tank culture apparatus, an airlift culture apparatus, a fluidized bed culture apparatus, a hollow fiber culture apparatus, a roller bottle culture apparatus, a packed bed culture apparatus, or a bag culture apparatus as the apparatus.

Another example of high expression of a protein includes high expression of a reporter gene for monitoring the behavior or biological functions of the protein of interest in mammalian cells. For example, a reporter gene (e.g., DNA encoding a fluorescent protein or a luminescent protein) linked to a gene control sequence (e.g., a promoter or an enhancer) can be operably inserted to, for example, the DNA of interest. High expression of the reporter gene can enhance the detection sensitivity of reporter assay. This enables the behavior of the DNA of interest expressed by certain stimulation in cells to be observed with florescence or luminescence as an index. Since such observation can usually be performed in laboratories, cells can be cultured using a solid medium depending on the type of the cells. In general, the observation can be performed using, for example, a fluorescence microscope with a camera.

When the protein is produced within host cells or on outer membranes of host cells, the desired protein can be aggressively secreted outside the host cells by use of the method of Paulson et al. [J. Biol. Chem., 264, 17619 (1989)], the method of Lowe et al. [Proc. Natl. Acad. Sci., USA, 86, 8227 (1989); and Genes Develop., 4, 1288 (1990)], or a method described in, for example, JP Patent Publication (Kokai) No. 05-336963 A (1993) or WO94/23021.

The amount of the desired protein produced may be elevated through the use of a gene amplification system using dihydrofolate reductase gene or the like (JP Patent Publication (Kokai) No. 2-227075 A (1990)).

In the collection step, for example, a supernatant or cells after centrifugation or filtration can be subjected to, for example, column chromatography such as affinity column chromatography, gel filtration chromatography, or ion exchange chromatography, filtration, ultrafiltration, salting out, dialysis, or a combination thereof, in order to obtain the desired protein produced by the culture. The recovery method is not limited to the methods described above and encompasses every method that may generally be used in protein purification.

Particularly, when the desired protein is a glycoprotein such as an antibody, the glycoprotein can be recovered using a protein A column, a protein G column, a protein L column, or an IgG-binding peptide column.

When the desired protein is expressed in a dissolved state in cells, the cells are recovered by centrifugation after the completion of culture, then suspended in an aqueous buffer solution, and disrupted using a sonicator, a French press, a Manton-Gaulin homogenizer, or Dyno-Mill to obtain cell-free extracts. From a supernatant obtained by the centrifugation of the cell-free extracts, a purified preparation can be obtained by using, singly or in combination, usual protein isolation or purification methods, i.e., approaches including solvent extraction, salting out with ammonium sulfate or the like, desalting, precipitation with an organic solvent, anion exchange chromatography using a resin such as diethylaminoethyl (DEAE)-Sepharose or DIAION HPA-75 (manufactured by Mitsubishi Chemical Corp.), cation exchange chromatography using a resin such as S-Sepharose FF (manufactured by Pharmacia), hydrophobic chromatography using a resin such as butyl-Sepharose or phenyl-Sepharose, gel filtration using a molecular sieve, affinity chromatography, chromatofocusing, and electrophoresis such as isoelectric focusing electrophoresis.

When the desired protein is expressed in an insoluble form in cells, the cells are recovered and then disrupted in the same manner as above, and centrifuged to recover the insoluble form of the protein as a precipitated fraction. The recovered insoluble form of the protein is solubilized with a protein denaturant. The solubilized solution is diluted or dialyzed so that the protein restores its normal conformation. Then, a purified polypeptide preparation can be obtained by the same isolation or purification methods as above.

When the desired protein or its derivative such as a sugar-modified form is secreted outside cells, the protein or its derivative such as a sugar-modified form can be recovered in a culture supernatant. The culture product is treated by an approach such as centrifugation in the same manner as above to obtain a soluble fraction. From the soluble fraction, a purified preparation can be obtained by use of the same isolation or purification methods as above.

### 5. Nonhuman animal carrying expression vector of present invention

The present invention further provides a nonhuman animal carrying the expression vector of the present invention. In the present specification, the term "nonhuman animal" includes, but not limited to, mammals, for example, primates, except for humans, such as monkeys and chimpanzees, rodents such as mice, rats, hamsters, and guinea pigs, and ungulates such as bovines, pigs, sheep, goats, horses, and camelids.

In the preparation of the nonhuman animal, the nonhuman animal can be obtained, for example, by transferring the expression vector to ES cells or iPS cells, injecting the cells into early embryo, transplanting the embryo into the uterus of a surrogate mother, and allowing the surrogate mother to give birth. Hereinafter, the preparation method will be described.

The ES cells can be established and maintained by isolating an inner cell mass from a blastocyst of a fertilized egg of an animal of interest and using mitomycin C-treated mouse embryonic fibroblast cells as a feeder (M.J. Evans and M.H. Kaufman, Nature 292: 154-156 (1981)).

The iPS cells form colonies in approximately 3 to 5 weeks by introducing certain reprogramming factors (DNA or proteins) into somatic cells (including somatic stem cells), and culturing and subculturing the cells in an appropriate medium. A known combination of the reprogramming factors is, for example, a combination of Oct3/4, Sox2, Klf4, and c-Myc; a combination of Oct3/4, Sox2, and Klf4; a combination of Oct4, Sox2, Nanog, and Lin28; or a combination of Oct3/4, Sox2, Klf4, c-Myc, Nanog, and Lin28 (K. Takahashi and S. Yamanaka, Cell 126: 663-676 (2006); WO2007/069666; M. Nakagawa et al., Nat. Biotechnol. 26: 101-106 (2008); K. Takahashi et al., Cell 131: 861-872 (2007); J. Yu et al., Science 318: 1917-1920 (2007); J. Liao et al., Cell Res. 18, 600-603 (2008)). Exemplary culture involves using a mitomycin C-treated mouse embryonic fibroblast cell line (e.g., STO) as feeder cells, and culturing somatic cells (approximately 10⁴ to 10⁵ cells/cm²) harboring the vector at a temperature of approximately 37°C using a medium for an ES cell on the feeder cell layer. The feeder cells are not always necessary (Takahashi, K. et al., Cell 131, 861-872, (2007)). Examples of the basic medium include Dulbecco's modified Eagle's medium (DMEM), Ham's F-12 medium, and mixed media thereof. For example, a medium for a mouse ES cell or a medium for a primate ES cell (Reprocell Inc.) can be used as the medium for an ES cell.

The ES cells and the iPS cells are known to contribute to the germline transmission. Therefore, the nonhuman animal (or a transgenic nonhuman animal) can be prepared by an approach involving injecting these cells harboring the expression vector of the present invention comprising the DNA of interest to early embryo of the same mammalian species as that from which the cells are derived, transplanting the embryo into the uterus of a surrogate mother, and allowing the surrogate mother to give birth. In addition, a male and a female of the obtained transgenic animals can be crossbred with each other to prepare homozygous animals and further, their offsprings.

In the case of using the nonhuman animal to produce, for example, a protein of different species (e.g., a human protein) encoded by the DNA of interest, an animal in which an endogenous gene corresponding to the DNA of interest has been deleted or completely inactivated can be used.

### 6. Gene and cell therapy with expression vector of present invention

Gene therapy can be performed by administering the expression vector of the present invention to a mammal, particularly, a human, and causing a protein encoded by the DNA of interest to be expressed in the body of the mammal.

Gene and cell therapy can be performed by administering or transplanting mammalian cells, particularly, human cells harboring the expression vector of the present invention, or cells or tissues prepared by the differentiation of pluripotent stem cells such as human iPS cells harboring the vector, and causing a protein encoded by the DNA of interest to be expressed in the body of the mammal.

Specifically, it is expected that an effect of gene and cell therapy is obtained by administering or transplanting the expression vector of the present invention comprising a disease causative gene or a therapeutic gene as the DNA of interest, or cells or tissues harboring the expression vector to a patient. For example, to a patient having, for example, cancer, muscular dystrophy, hemophilia, a neurodegenerative disease, an autoimmune disease, an allergic disease, or a genetic disease, the expression vector of the present invention comprising a causative gene or a therapeutic gene of these diseases (for example, chimeric antigen receptor (CAR) ) or cells or tissues harboring the expression vector is administered or transplanted to the patient. As a result, a therapeutic effect brought about by the transferred gene can be expected.

In Examples described below, HAC vector was constructed which had the promoter activation sequence of the present invention (2549 bp: SEQ ID NO: 1) at 5' upstream of a promoter (UBCP promoter, EF1α promoter, and PGK promoter) and had an indicator gene (red fluorescent protein gene or orange fluorescent protein) at 3' downstream of the promoter. The HAC vector was transferred to CHO cells or human mesenchymal cells, and the promoter activation sequence of the present invention was evaluated for its anti-silencing effect and activity manifestation enhancing effect with the expression of the indicator protein as an index. As a result, the promoter activation sequence of the present invention was demonstrated to actually have the anti-silencing effect and the activity manifestation enhancing effect.

### Examples

The present invention will be described further specifically with reference to Examples described below. However, the technical scope of the present invention is not limited by these Examples.

### [Example 1] Construction of red fluorescent protein (mCherry) expression vector for human artificial chromosome (HAC) transfer

An additional sequence element was studied for its anti-silencing effect and activity enhancing effect on a generallu used promoter in a CHO hprt-deficient (hprt-) cell line carrying HAC vector (21HAC2) (21HAC2/CHOhprt-; Kazuki Y et al., Gene Ther 18 (4): 384-93, 2011). 21HAC2/CHOhprt- is cotransfected with a vector obtained from I-EGFP-I-loxP-3'HPRT vector (Figure 2; Kazuki Y et al., Gene Ther 18 (4): 384-93, 2011) by the substitution of a CAG promoter-EGFP-polyA moiety by each of other various expression units, and a Cre expression vector (Kazuki Y et al., Gene Ther 18 (4): 384-93, 2011) to obtain HAT-resistant clones with one copy of the expression unit inserted in 21HAC2 (Figure 2).

### 1. Construction of pUms vector

A fragment comprising HS4 (chicken insulator sequence element) and NotI/SalI/SpeI was amplified with the I-EGFP-I-loxP-3'HPRT vector as a template. PCR reaction of 35 cycles each involving 98°C for 10 seconds --> 68°C for 4 minutes was performed using primers given below and Primestar GXL premix (Takara Bio Inc.). The I-EGFP-I-loxP-3'HPRT vector was digested with restriction enzymes NcoI (NEB) and SpeI (NEB), and the amplified fragment was inserted thereto to prepare X3.1-MCS vector (Figure 3). An expression unit composed of human ubiquitin C core promoter (approximately 1.2 kb; Schorpp M et al. Nucleic Acids Res. 24 (9):1787-1788, 1996), a coding region of red fluorescent protein mCherry (Nathan C et al., Nat Biotechnol 22 (12): 1567-72, 2004) gene, and a SV40 polyA sequence was designed such that NotI sites were located at both ends thereof. The resulting sequence was synthesized at VectorBuilder Inc. A UbCP-mCherry-SV40pA sequence (NotI fragment) derived from the synthetic DNA was inserted to the X3.1-MCS vector digested with NotI (NEB) to prepare X3.1-UbCP-mCherry-SV40pA (Figure 3; hereinafter, referred to as pUms vector).
HS4-F2: 5'-ATCCATGGATCGACTCTAGAGGGACAGCC-3' (SEQ ID No. 3)
HS4-R1: 5'-ATAACTAGTCGACGCGGCCGCCTCACTGACTCCGTCCTGGA-3' (SEQ ID No. 4)

### 2. Construction of mCherry expression vector with genomic sequence upstream of UbC gene added to pUms vector

Vectors for HAC transfer having an expression unit of UbCP/mCherry gene/SV40 polyA sequence to which a region of approximately 2.5 kb (Figure 1) or a partial fragment thereof (0.8 kb; Figure 1) in a genomic sequence of approximately 15 kb (hereinafter, referred to as UL15) at 5' upstream of human UbC gene, were added respectively, were prepared by the following procedures (referred to as pUL0.8ms vector and pUL2.5ms vector, respectively). These two genomic sequences at 5' upstream of the human UbC gene were each prepared by PCR amplification with a human chromosomal BAC clone RP11-592O2 as a template using primers given below. PCR reaction of 5 cycles each involving 98°C for 10 seconds --> 72°C for 10 minutes, 5 cycles each involving 98°C for 10 seconds -> 70°C for 10 minutes, and 30 cycles each involving 98°C for 10 seconds -> 68°C for 10 minutes was performed using primers given below and Primestar GXL premix (Takara Bio Inc.). The amplified fragment was further digested with SalI and KpnI, and the obtained fragment was cloned into the pUms vector digested with SalI and KpnI. The construction of the vectors is summarized in Figure 4.
pUL0.8ms-F: 5'-CATGTCGACCCAGAGACAACCACAGAACC-3' (SEQ ID No. 5)
pUL0.8ms-R: 5'-AGTGGTACCCGCACGCAGTCCCTGCTGAA-3' (SEQ ID No. 6)
pUL2.5ms-F: 5'-CATGTCGACACCCGGAAGGCGGAGGTTAC-3' (SEQ ID No. 7)
pUL2.5ms-R: 5'-AGTGGTACCAGGAAGTTCAGGAAATTGGC-3' (SEQ ID No. 8)

### 3. Construction of pSUL15ms vector

A vector for HAC transfer having an expression unit of UbCP/mCherry gene/SV40 polyA sequence with UL15 added was prepared by the following procedures. pSTV28 vector (Takara Bio Inc.) was digested with EcoRI and XbaI, and a DNA fragment comprising AscI and SmaI sites prepared by the annealing of the following synthetic DNA sequences was inserted thereto to prepare pSTV28+MCS vector (Figure 5).
pSTV28-MCS_1: 5'-CTAGACCCGGGATTGGCGCGCCG-3' (SEQ ID No. 9)
pSTV28-MCS_2: 5'-AATTCGGCGCGCCAATCCCGGGT-3' (SEQ ID No. 10)

Vectors for HAC transfers having an expression unit of UbCP/mCherry gene/SV40 polyA sequence to which a region of approximately 4 kb proximal to a UBC gene coding region and a region of approximately 11 kb distal to the UBC gene coding region in a genomic sequence of approximately 15 kb (hereinafter, referred to as UL15) at 5' upstream of human UbC gene were added respectively, were prepared by the method as described in 2. (referred to as pUL4ms vector and pUL11ms vector, respectively). These two genomic sequences at 5' upstream of the human UbC gene were each prepared by PCR amplification with a human chromosomal BAC clone RP11-592O2 as a template using primers given below. PCR reaction of 5 cycles each involving 98°C for 10 seconds --> 72°C for 10 minutes, 5 cycles each involving 98°C for 10 seconds -> 70°C for 10 minutes, and 30 cycles each involving 98°C for 10 seconds -> 68°C for 10 minutes was performed using primers given below and Primestar GXL premix (Takara Bio Inc.). The amplified fragment was further digested with SalI and KpnI, and the obtained fragment was cloned into the pUms vector digested with SalI and KpnI.
pUL4ms-F: 5'-ACTGGTACCATCGATTCAGCCAGGCACATTAGCTC-3' (SEQ ID No. 11)
pUL4ms-R: 5'-GAGGTCGACCGGGAGCGGGTGGGCGGCCC-3' (SEQ ID No. 12)
pUL11ms-F: 5'-TATGTCGACATCGATATACAGACATGAGCTAATGT-3' (SEQ ID No. 13)
pUL11ms-F: 5'-AGTGGTACCAGGAAGTTCAGGAAATTGGC-3' (SEQ ID No. 14)

A DNA fragment between the AscI and SmaI sites of the pSTV28+MCS vector was substituted by a DNA fragment between the Seal and AscI sites comprising UL4-UbCP-mCherry-SV40pA of the pUL4ms vector to prepare pSTV-UL4-UbCP-mCherry-SV40pA (Figure 5; hereinafter, referred to as pSUL4ms vector). The pSUL4ms vector was digested with KpnI and ClaI, and a DNA fragment between the KpnI and ClaI sites comprising a UL11 sequence of the pUL11ms vector was inserted thereto to prepare pSTV-UL15-UbCP-mCherry-SV40pA (Figure 6; hereinafter, referred to as pSUL15ms vector).

### [Example 2] Study on anti-silencing effect and activity enhancing effect of additional sequence element on UBCP-mcherry expression unit in CHO cell

The pUms vector or the pUL2.5ms vector prepared in Example 1 was inserted to 21HAC2 vector in 21HAC2/CHOhprt- cells. The expression vector was transferred together with a recombinase (Cre) expression vector pBS185 (Kazuki Y et al., Gene Ther 18 (4): 384-93, 2011) to the 21HAC2/CHOhprt- cells carrying the 21HAC2 vector by the electrical pulse method using NEPA21 (Nepa Gene Co., Ltd., 150 V, 5.0 msec). After transfer by electrical pulse, the cells were inoculated to one 10 cm dish and 48 hours later, cultured in Ham's F12 medium containing 2% HAT (Sigma-Aldrich Co., LLC) and 10% FBS (Nichirei Corp.) to obtain HAT-resistant colonies. The obtained HAT-resistant cell populations were subjected to FACS analysis after 7 weeks from transfer and evaluated for the expression of the red fluorescent protein. The results are shown in Figure 7. A mean median value (4302) of positive peaks of the HAT-resistant cell population harboring the pUL2.5ms vector was higher than a mean median value (1662) of positive peaks of the cell population harboring the pUms vector. This indicated that the addition of the fragment of 2.5 kb to UBCP brings about a promoter activity enhancing effect.

### [Example 3] Study on anti-silencing effect and activity enhancing effect of additional sequence element on UBCP-mcherry expression unit in human mesenchymal stem cell

The pUms vector, the pUL0.8ms vector, or the pUL2.5ms vector prepared in Example 1 was inserted to 21HAC2 vector in a hprt- line of 21HAC2/human immortalized mesenchymal stem cells (Okamoto T et al., BBRC 295: 354-361, 2002). The expression vector was transferred together with a recombinase (Cre) expression vector pBS185 (Kazuki Y et al., Gene Ther 18 (4): 384-93, 2011) to the 21HAC2/hiMSChprt- cells carrying the 21HAC2 vector by the electrical pulse method using NEPA21 (Nepa Gene Co., Ltd., 150 V, 5.0 msec). After transfer by electrical pulse, the cells were inoculated to a 10 cm dish and 48 hours later, cultured in DMEM medium containing 2% HAT (Sigma-Aldrich Co., LLC) and 10% FBS (Nichirei Corp.) to obtain HAT-resistant colonies. The obtained HAT-resistant cells were subjected to microscopic observation of the expression of the red fluorescent protein after 2 weeks from transfer. The results are shown in Figure 8, together with bright field images. Red fluorescence intensity in the cells harboring the pUL2.5ms vector was evidently strong as compared with red fluorescence intensity in the cells harboring the pUms vector or the pUL0.8ms vector. This indicated that: the addition of the fragment of 2.5 kb to UBCP brings about a promoter activity enhancing effect; and the addition of the fragment of approximately 0.8 kb lacks this effect.

### [Example 4] Construction of orange fluorescent protein (Kusabira Orange: KO) expression vector for human artificial chromosome (HAC) transfer

φC31 attB-NeoR-pUbc-KO1 vector (described in Kazuki Y et al., Molecular Therapy: Nucleic Acids 23: 629-639, 2021) comprises an expression unit composed of human ubiquitin C core promoter (approximately 1.2 kb; Schorpp M et al., Nucleic Acids Res. 24 (9): 1787-1788, 1996), a coding region of orange fluorescent protein KO (MBL) gene, and a bovine growth hormone gene-derived polyA sequence. Basal-HAC/CHO cells are cotransfected with the vector and a φC31 integrase expression vector (Kazuki Y et al., Molecular Therapy: Nucleic Acids 23: 629-639, 2021) to obtain G418-resistant clones with one copy of the expression unit inserted in Basal-HAC (Kazuki Y et al., Molecular Therapy: Nucleic Acids 23: 629-639, 2021).

First, the φC31 attB-NeoR-pUbc-KO1 vector was blunt-ended by digestion with KpnI and self-ligated to prepare φC31 attB-NeoR-pUbc-KO1(KpnI-) vector (Figure 9). Subsequently, a DNA fragment comprising SalI and KpnI sites (multicloning sites) prepared by the annealing of the following sequences was inserted to a SalI site upstream of UbC promoter to construct pUbC-hKO+MCS vector (hereinafter, referred to as pUL vector; Figure 9).
Fw.XhoI-KpnI-SalI: 5'-TCGAGAGTCGGTACCATGCG-3' (SEQ ID No. 15)
XhoI-KpnI-SalI: 5'-TCGACGCATGGTACCGACTC-3' (SEQ ID No. 16)

A vector with a genomic sequence of approximately 2.5 kb at 5' upstream of human UbC gene added to UBCP of the pUL vector was further constructed in the same manner as in Example 1 (Figure 9; referred to as pUL2.5 vector).

A DNA fragment between the PvuI and SacI sites of the pSTV28+MCS vector prepared in Example 1 was substituted by a DNA fragment between the PvuI and SacI sites comprising UbC-KO-BGHpA of the pUL vector to prepare pSTV-UbC-hKO-BGHpA vector (hereinafter, referred to as pSUkb vector; Figure 10). The pSUkb vector was digested with SalI and KpnI, and a DNA fragment comprising the genomic sequence of approximately 15 kb at 5' upstream of the UbC gene between the SalI and KpnI sites of the pSUL15ms vector was inserted thereto to prepare pSUL15 vector (Figure 10).

### [Example 5] Study on anti-silencing effect and activity enhancing effect of additional sequence element on UBCP-KO expression unit in CHO cell

The pUL vector, the pUL2.5 vector, or the pSUL15 vector prepared in Example 4 was inserted to Basal-HAC vector in Basal-HAC/CHO cells. The expression vector was transferred together with a recombinase (φC31 integrase) expression vector (Kazuki Y et al., Molecular Therapy: Nucleic Acids 23: 629-639, 2021) to the Basal-HAC/CHO cells by the electrical pulse method using NEPA21 (Nepa Gene Co., Ltd., 150 V, 5.0 msec). After transfer by electrical pulse, the cells were inoculated to a 96-well plate and 48 hours later, cultured in Ham's F12 medium containing G418 (FUJIFILM Wako Pure Chemical Corp.) and 10% FBS (Nichirei Corp.), and G418-resistant colonies were isolated and expansion-cultured. Five or more clones were selected for each vector, subjected to FACS analysis, and evaluated for the expression of the orange fluorescent protein. The results are shown in Figure 11. A mean median value of positive peaks of 6 clones having the pUL vector was 1300, whereas the mean was 14400 and 14000 for the pUL2.5 vector and pUL15 vector, respectively. Two out of the 6 clones having the pUL vector had a higher ratio of a negative population than that of a positive population, whereas no negative population was observed in all the 6 clones having the pUL2.5 vector or the pSUL15 vector. This indicated that the addition of the fragment of 2.5 kb brings about expression enhancing activity and anti-silencing activity at a level equivalent to that of the addition of the fragment of 15 kb.

### [Example 6] Study on anti-silencing effect and activity enhancing effect of additional sequence element on UBCP-KO expression unit in human iPS cell

The pUL vector or the pUL2.5 vector prepared in Example 4 was inserted to Basal-HAC vector in Basal-HAC/human iPS cells (Kazuki Y et al., Molecular Therapy: Nucleic Acids 23: 629-639, 2021). The expression vector was transferred together with a recombinase (φC31 integrase) expression vector (Kazuki Y et al., Molecular Therapy: Nucleic Acids 23: 629-639, 2021) to the Basal-HAC/human iPS cells by the electrical pulse method. After transfer by electrical pulse, the cells were inoculated to a 96-well plate and 48 hours later, cultured in StemFit medium (Takara Bio Inc.) containing G418 (90 µg/mL), and G418-resistant colonies were isolated and expansion-cultured. 12 or more single clones were selected for each vector, subjected to FACS analysis, and evaluated for the expression of the orange fluorescent protein Kusabira Orange (KO). Results of evaluating KO fluorescence intensity in the clones harboring each vector are shown in Figure 20. As a result, the ratio of a KO-positive population obtained by the transfer of the pUL2.5 vector was high as compared with the case of transferring the pUL vector, indicating that the ratio of KO-expressing cells is increased in human iPS cells, owing to the anti-silencing effect and the activity enhancing effect of the fragment of 2.5 kb on UBCP.

### [Example 7] Construction of mCherry expression vector using EF1A promoter with genomic sequence upstream of UbC gene added

An expression unit composed of human elongation factor 1α1 (EF1α) gene promoter (EF1AP; approximately 1.2 kb; Qin JY et al., PLoS One. 5 (5): e10611 2010), a coding region of red fluorescent protein mCherry gene, and a SV40 polyA sequence was designed such that NotI sites were located at both ends thereof. The resulting sequence was synthesized at VectorBuilder Inc. An EF1A-mCherry-SV40pA sequence (NotI fragment) derived from the synthetic DNA was inserted to the X3.1-MCS vector digested with NotI in the same manner as in Example 1 to prepare X3.1-EF1A-mCherry-SV40pA (hereinafter, referred to as pEFms vector; Figure 12). A vector for HAC transfer having an expression unit of EF1AP/mCherry gene/SV40 polyA sequence with a genomic sequence of approximately 2.5 kb at 5' upstream of human UbC gene added was prepared in the same manner as in Example 1 (hereinafter, referred to as pEF2.5ms vector; Figure 12).

### [Example 8] Study on anti-silencing effect and activity enhancing effect of additional sequence element on EF1AP-mcherry expression unit in CHO cell

The pEFms vector or the pEF2.5ms vector prepared in Example 7 was inserted to 21HAC2 vector in 21HAC2/CHOhprt- cells. The expression vector was transferred together with a recombinase (Cre) expression vector pBS185 (Kazuki Y et al., Gene Ther 18 (4): 384-93, 2011) to the 21HAC2/CHOhprt- cells carrying the 21HAC2 vector by the electrical pulse method using NEPA21 (Nepa Gene Co., Ltd., 150 V, 5.0 msec). After transfer by electrical pulse, the cells were inoculated to one 10 cm dish and 48 hours later, cultured in Ham's F12 medium containing 2% HAT (Sigma-Aldrich Co., LLC) and 10% FBS (Nichirei Corp.) to obtain HAT-resistant colonies. The obtained HAT-resistant cell populations were subjected to FACS analysis after 4 weeks and 7 weeks from transfer and evaluated for the expression of the red fluorescent protein. The results are shown in Figure 13. The HAT-resistant cell population harboring the pEFms vector had a higher ratio of a mcherry-negative population than that of a positive population, whereas almost no negative population was observed in the cell population harboring the pUL2.5 vector. This indicated that the addition of the fragment of 2.5 kb to EF1AP brings about an anti-silencing effect.

### [Example 9] Construction of mCherry expression vector using PGK promoter with genomic sequence upstream of UbC gene added

An expression unit composed of human phosphoglycerate kinase 1(PGK) gene promoter (PGKP; approximately 0.5 kb; Kita-Matsuo H et al., PLoS One. 4 (4): e5046. 2009), a coding region of red fluorescent protein mCherry gene, and a SV40 polyA sequence was designed such that NotI sites were located at both ends thereof. The resulting sequence was synthesized at VectorBuilder Inc. A PGKP-mCherry-SV40pA sequence (NotI fragment) derived from the synthetic DNA was inserted to the X3.1-MCS vector digested with NotI in the same manner as in Example 1 to prepare X3.1-PGKP-mCherry-SV40pA (hereinafter, referred to as pPGKms vector; Figure 12). A vector for HAC transfer having an expression unit of PGKP/mCherry gene/SV40 polyA sequence with a genomic sequence of approximately 2.5 kb at 5' upstream of human UbC gene added was prepared in the same manner as in Example 1 (hereinafter, referred to as pPGK2.5ms vector; Figure 12).

### [Example 10] Study on anti-silencing effect and activity enhancing effect of additional sequence element on PGKP-mcherry expression unit in CHO cell

The pPGKms vector, the pPGK2.5ms vector, or the pPGK4ms vector prepared in Example 9 was inserted to 21HAC2 vector in 21HAC2/CHOhprt- cells. The expression vector was transferred together with a recombinase (Cre) expression vector pBS185 (Kazuki Y et al., Gene Ther 18 (4): 384-93, 2011) to the 21HAC2/CHOhprt- cells carrying the 21HAC2 vector by the electrical pulse method using NEPA21 (Nepa Gene Co., Ltd., 150 V, 5.0 msec). After transfer by electrical pulse, the cells were inoculated to one 10 cm dish and 48 hours later, cultured in Ham's F12 medium containing 2% HAT (Sigma-Aldrich Co., LLC) and 10% FBS (Nichirei Corp.) to obtain HAT-resistant colonies. The obtained HAT-resistant cell populations were subjected to FACS analysis after 4 weeks and 7 weeks from transfer and evaluated for the expression of the red fluorescent protein. The results are shown in Figure 14. The HAT-resistant cell population harboring the pPGKms vector had a higher ratio of a mcherry-negative population than that of a positive population. On the other hand, almost no negative population was observed in the cell population harboring the pPGK2.5ms vector. This indicated that the addition of the fragment of 2.5 kb to EF1AP brings about an anti-silencing effect.

### [Example 11] Study on anti-silencing effect and activity enhancing effect of additional sequence element on PGKP-mcherry expression unit in human mesenchymal stem cell

The pPGKms vector or the pPGK2.5ms vector prepared in Example 9 was inserted to 21HAC2 vector in 21HAC2/CHOhprt- cells. The expression vector was transferred together with a recombinase (Cre) expression vector pBS185 (Kazuki Y et al., Gene Ther 18 (4): 384-93, 2011) to the 21HAC2/hiMSChprt- cells carrying the 21HAC2 vector by the electrical pulse method using NEPA21 (Nepa Gene Co., Ltd., 150 V, 5.0 msec). After transfer by electrical pulse, the cells were inoculated to a 10 cm dish and 48 hours later, cultured in DMEM medium containing 2% HAT (Sigma-Aldrich Co., LLC) and 10% FBS (Nichirei Corp.) to obtain HAT-resistant colonies. The obtained HAT-resistant cell populations were subjected to FACS analysis after 4 weeks from transfer and evaluated for the expression of the red fluorescent protein. The results are shown in Figure 15. The HAT-resistant cell population harboring the pPGKms vector had a higher ratio of a mcherry-negative population than that of a positive population. On the other hand, almost no negative population was observed in the cell population harboring the pPGK2.5ms vector. This indicated that the addition of the fragment of 2.5 kb to PGKP brings about an anti-silencing effect.

### [Example 12] Construction of mCherry expression vector for random insertion with genomic sequence upstream of UbC gene added

A vector for random insertion having an expression unit of UbCP/mCherry-NeoR (neomycin-resistant) fusion gene/SV40 polyA sequence to which a region of approximately 2.5 kb (Figure 1) in a genomic sequence of approximately 15 kb at 5' upstream of human UbC gene was added was prepared by the following procedures (referred to as pUL2.5mNs vector).

A fragment with SpeI and FseI sites added to both ends of mCherry-NeoR was amplified by PCR with mCherry_NeoR-CMV_Luciferase vector synthesized at VectorBuilder Inc. as a template using primers described below. PCR reaction of 35 cycles each involving 98°C for 10 seconds --> 60°C for 5 seconds --> 68°C for 5 seconds was performed using primers described below and KOD One PCR Master Mix (Toyobo Co., Ltd.). The pUL2.5ms vector prepared in (Example 1) was digested with SpeI and FseI, and the mCherry-NeoR amplified fragment digested with SpeI and FseI was inserted thereto to prepare pUL2.5mNs vector. The configuration of the pUL2.5mNs vector is summarized in Figure 16.
SpeI_ mCherry Fw: 5'-TAGACTAGTAAATTGTCCGCTAAATTCTGGCCGTTTTTGGCTTTTTTGTTAGACAA TGGTGAGCAAGGGCGAGGAGGA-3' (SEQ ID No. 17)
FseI_NeoR Rv: 5'-CTTAGGCCGGCCACTCGAGTCAGAAGAACTCGTCAAGAAGGCG-3' (SEQ ID No. 18)

pUmNs vector obtained by the deletion of the region of approximately 2.5 kb from the pUL2.5mNs vector was prepared by digesting the pUL2.5mNs vector with SalI and KpnI, followed by blunt ending using Blunting Kit (Takara Bio Inc.). The structure of the pUmNs vector is summarized in Figure 17.

### [Example 13] Study on anti-silencing effect and activity enhancing effect of additional sequence element on UbCP-mCherry-NeoR expression unit in gene transfer by random insertion to CHO

The pUmNs vector or the pUL2.5mNs vector prepared in Example 12 was transferred to 21HAC2/CHOhprt- cells. Unlike Example 2, etc., a recombinase (Cre) expression vector was not transferred at the same time therewith. This produces neomycin (G418) resistance when the transferred vector is randomly inserted to the genome of CHO cells. The vector was digested with a restriction enzyme Seal, then recovered by the ethanol precipitation method, and transferred to 21HAC2/CHOhprt- by the electrical pulse method using NEPA21 (Nepa Gene Co., Ltd., 150 V, 5.0 msec). After transfer by electrical pulse, the cells were inoculated to two 10 cm dishes and 48 hours later, cultured in Ham's F12 medium containing 800 µg/mL G418 (FUJIFILM Wako Pure Chemical Corp.) and 10% FBS (Nichirei Corp.) to obtain drug-resistant colonies. A mixed cell population of the obtained drug-resistant colonies was subjected to FACS analysis after 2 weeks from transfer, and red fluorescence intensity was compared. The results are shown in Figure 18. In the G418-resistant cells having the pUmNs vector, the number of mCherry-positive (fraction P7) cells based on the total number of cells was 1136 (5.5%) out of 20826 cells. In the G418-resistant cells having the pUL2.5mNs vector, the number of mCherry-positive (fraction P7) cells based on the total number of cells was 1481 (11.8%) out of 12595 cells. The ratio of a strongly mCherry-positive fraction to the total number of cells was evaluated on 2 weeks, 4 weeks, or 7 weeks from transfer by the FACS analysis of the drug-resistant cell population. The results are shown in Figures 21-1 and 21-2. A portion enclosed by the thick-line box in Figure 21-1 depicts a strongly mCherry-positive fraction (P6). Figure 21-2 shows results of conducting FACS analysis as to G418-resistant cells on 2 weeks, 4 weeks, or 7 weeks from transfer by electrical pulse, and measuring the ratio of a strongly mCherry-positive fraction (P6) to the total number of cells. In three independent gene transfer experiments, the ratio of cells in a strongly mCherry-positive fraction (P6) that exhibited fluorescence intensity of 1000 or more was 10% or more in all the G418-resistant cells of week 4 and week 7 having the pUL2.5mNs vector (mean on week 4: 15.87%, mean on week 7: 13.10%) and by contrast, was 0.1% or less in all the G418-resistant cells having the pUmNs vector. Thus, the ratio of the mcherry-positive population harboring the pUL2.5mNs vector was high as compared with the case of transferring the pUmNs vector, indicating that the ratio of mcherry-expressing cells is increased in human iPS cells, owing to the anti-silencing effect and the activity enhancing effect of the fragment of 2.5 kb on UBCP.

### [Example 14] Study on anti-silencing effect and activity enhancing effect of additional sequence element on UbCP-mCherry-NeoR expression unit in gene transfer by random insertion to human iPS cell

The pUmNs vector or the pUL2.5mNs vector prepared in Example 12 was transferred to human iPS cells (201B7: Riken Cell Bank No. HPS0063). Unlike Example 2, etc., a recombinase (Cre) expression vector was not transferred at the same time therewith. This produces neomycin (G418) resistance when the transferred vector is randomly inserted to the genome of CHO cells. The vector was digested with a restriction enzyme Seal, then recovered by the ethanol precipitation method, and transferred to 201B7 by the electrical pulse method using NEPA21 (Nepa Gene Co., Ltd., 175 V, 2.5 msec). After transfer by electrical pulse, the cells were inoculated to two 10 cm dishes and 48 hours later, cultured in StemFit medium (Takara Bio Inc.) containing 70 µg/mL G418 (FUJIFILM Wako Pure Chemical Corp.) to obtain drug-resistant colonies. One of the 10 cm dishes was treated with methanol for 15 minutes after 7 days from transfer, and stained with CBB staining solution (0.25% CBB/10% ethanol/40% methanol) for 15 minutes. The 10 cm dish thus stained was photographed, and the number of colonies was measured and compared. As a result, the rate of appearance of drug-resistant colonies was increased owing to the anti-silencing effect and the activity enhancing effect of the fragment of 2.5 kb on UBCP (Figure 19). The cells in the other 10 cm dish were cultured by changing the G418 concentration to 50 µg/ml 8 days or later after transfer, and subjected to FACS analysis after 2 weeks from transfer, and red fluorescence intensity was compared. The results indicate that the ratio of mcherry-expressing cells is increased owing to the anti-silencing effect and the activity enhancing effect of the fragment of 2.5 kb on UBCP.

### [Example 15] Construction of mCherry expression vector for random insertion using EF1A promoter with genomic sequence upstream of UbC gene added

The vector for HAC transfer comprising an expression unit of EF1AP/red fluorescent protein mCherry gene coding region/SV40 polyA sequence (pEFms vector; the top vector of Figure 12), and the vector for HAC transfer having an expression unit of EF1AP/mCherry gene/SV40 polyA sequence with a genomic sequence of approximately 2.5 kb at 5' upstream of human UbC gene added (pEF2.5ms vector; the second top vector of Figure 12), constructed in Example 7 were each used as a starting material, and pEFmNs vector (Figure 22) and pEF2.5mNs vector (Figure 22) were constructed by substituting the mCherry gene by the mCherry-NeoR (neomycin-resistant) fusion gene shown in [Example 12].

### [Example 16] Study on anti-silencing effect and activity enhancing effect of additional sequence element on EF1A-mCherry-NeoR expression unit in gene transfer by random insertion to CHO cell

The pEFmNs vector or the pEF2.5mNs vector prepared in Example 15 was transferred to 21HAC2/CHOhprt- cells in the same manner as in Example 13. Unlike Example 2, etc., a recombinase (Cre) expression vector was not transferred at the same time therewith. This produces neomycin (G418) resistance when the transferred vector is randomly inserted to the genome of CHO cells. The vector was digested with a restriction enzyme Seal, then recovered by the ethanol precipitation method, and transferred to, 21HAC2/CHOhprt- by the electrical pulse method using NEPA21 (Nepa Gene Co., Ltd., 150 V, 5.0 msec). After transfer by electrical pulse, the cells were inoculated to two 10 cm dishes and 48 hours later, cultured in Ham's F12 medium containing 800 µg/mL G418 (FUJIFILM Wako Pure Chemical Corp.) and 10% FBS (Nichirei Corp.) to obtain drug-resistant colonies. A mixed cell population of the obtained drug-resistant colonies was subjected to FACS analysis after 4 weeks from transfer, and red fluorescence intensity was compared. The results are shown in Figure 23. Results of three independent experiments are shown as to the pEFmNs vector, and results of two independent experiments are shown as to the pEF2.5mNs vector. The G418-resistant cells having the pEFmNs vector had median mCherry fluorescence intensity of 649, 828, and 509, respectively, and a mCherry-positive cell ratio of 67%, 77.42%, and 62.56%, respectively, in the total number of cells. On the other hand, the G418-resistant cells having pEF2.5mNs vector had median mCherry fluorescence intensity of 2986 and 2625, respectively, and a mCherry-positive cell ratio of 94.15% and 89.22%, respectively, in the total number of cells. Thus, the ratio of the mcherry-positive population harboring the pEF2.5mNs vector was high as compared with the case of transferring the pEFmNs vector, indicating that the ratio of mcherry-expressing cells is increased owing to the anti-silencing effect and the activity enhancing effect of the fragment of 2.5 kb on the EF1A promoter.

### Industrial Applicability

A mammalian cell, having an expression vector of the present invention comprising a promoter activation sequence, a promoter, and a DNA of interest, is prepared. The mammalian cell is useful for the purpose of stably and efficiently production of a protein encoded by the DNA of interest in the mammalian cell.

### Free Text of Sequence Listing

SEQ ID NOs: 3 to 8: Primer
SEQ ID NOs: 9 and 10: Annealing sequence for preparing a DNA fragment comprising an AscI site and a SmaI site
SEQ ID NOs: 11 to 14: Primer
SEQ ID NOs: 15 and 16: Annealing sequence for preparing a DNA fragment comprising a SalI site and a KpnI site
SEQ ID NOs: 17 and 18: Primer

All publications, patents, and patent applications cited herein are incorporated herein by reference in their entirety.

## Claims

1. A promoter activation sequence comprising a nucleotide sequence represented by SEQ ID NO: 1 or a nucleotide sequence of 850 or more in a nucleotide sequence having 85% or higher sequence identity thereto.

2. An expression vector comprising the promoter activation sequence according to claim 1, a promoter, and a DNA of interest.

3. The expression vector according to claim 2, wherein the promoter is a constitutive expression promoter.

4. The expression vector according to claim 3, wherein the constitutive expression promoter is selected from the group consisting of EF1α promoter, PGK promoter, and UBC promoter.

5. The expression vector according to any one of claims 2 to 4, wherein the promoter activation sequence according to claim 1 is comprised at 5' upstream of the promoter.

6. The expression vector according to any one of claims 2 to 5, wherein the expression vector is a mammalian artificial chromosome vector.

7. The expression vector according to claim 6, wherein the expression vector is a human artificial chromosome vector.

8. A mammalian cell carrying the expression vector according to any one of claims 2 to 7.

9. The cell according to claim 8, wherein the animal cell is a human cell or a CHO cell.

10. The cell according to claim 9, wherein the human cell is a human pluripotent stem cell or a human somatic stem cell.

11. The cell according to claim 10, wherein the human pluripotent stem cell is a human iPS cell or a human ES cell, and the human somatic stem cell is a human MSC cell.

12. A nonhuman mammal comprising the expression vector according to any one of claims 2 to 7.

13. A method for producing a protein encoded by a DNA of interest, comprising culturing the mammalian cell according to any one of claims 8 to 11 in a medium for animal cell culture, and collecting the protein encoded by the DNA of interest from a culture product obtained by the culture.
